(19) Europäisches Patentamt
European Patent Office
Office européen des brevets

(11) EP 0 580 008 B1

(12) **EUROPÄISCHE PATENTSCHRIFT**

(45) Veröffentlichungstag und Bekanntmachung des Hinweises auf die Patenterteilung:
**08.07.1998 Patentblatt 1998/28**

(51) Int. Cl.$^6$: **C07K 5/10**, C07D 233/40, C07D 233/42, A61K 38/04, A61K 31/415, C07K 5/08

(21) Anmeldenummer: **93110754.4**

(22) Anmeldetag: **06.07.1993**

(54) **Phenylimidazolidin-derivate, ihre Herstellung und ihre Verwendung**
Phenylimidazolidine Derivatives, their preparation and use
Dérivés de la phénylimidazolidine, leur préparation et utilisation

(84) Benannte Vertragsstaaten:
**AT BE CH DE DK ES FR GB IT LI NL SE**

(30) Priorität: **24.07.1992 DE 4224414**

(43) Veröffentlichungstag der Anmeldung:
**26.01.1994 Patentblatt 1994/04**

(73) Patentinhaber:
**HOECHST AKTIENGESELLSCHAFT
65926 Frankfurt am Main (DE)**

(72) Erfinder:
- **Zoller, Gerhard, Dr.
D-61137 Schöneck (DE)**
- **Jablonka, Bernd, Dr.
D-65812 Bad Soden (DE)**
- **Just, Melitta, Dr.
D-63225 Langen (DE)**
- **Klingler, Otmar, Dr.
D-63110 Rodgau (DE)**
- **Breipohl, Gerhard, Dr.
D-60529 Frankfurt am Main (DE)**
- **Knolle, Jochen, Dr.
D-65830 Kriftel (DE)**
- **König, Wolfgang, Dr.
D-94375 Stallwang (DE)**

(56) Entgegenhaltungen:
**EP-A- 0 530 505**

- **CHEMICAL ABSTRACTS, vol. 109, no. 9, 29. August 1988, Columbus, Ohio, US; abstract no. 66704n, N CHATTERJIE ET AL. 'Hydantoin derivatives of amantadine; anticonvulsive effects in mice' Seite 48 ;**

Bemerkungen:
Die Akte enthält technische Angaben, die nach dem Eingang der Anmeldung eingereicht wurden und die nicht in dieser Patentschrift enthalten sind.

EP 0 580 008 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

## Beschreibung

Die vorliegende Erfindung betrifft Phenylimidazolidin-derivate, ihre Herstellung und ihre Verwendung als Hemmstoffe der Blutplättchenaggregation.

In der EP-A 449 079, sowie in der noch nicht veröffentlichten deutschen Patentanmeldung P 41 26 277.8 sind Hydantoinderivate mit thrombozytenaggregationshemmender Wirkung beschrieben. Weitere Forschungsarbeiten haben gezeigt, daß auch die Verbindungen der vorliegenden Erfindung starke Hemmstoffe der Blutplättchenaggregation sind.

Die vorliegenden Erfindung betrifft Verbindungen der allgemeinen Formel I,

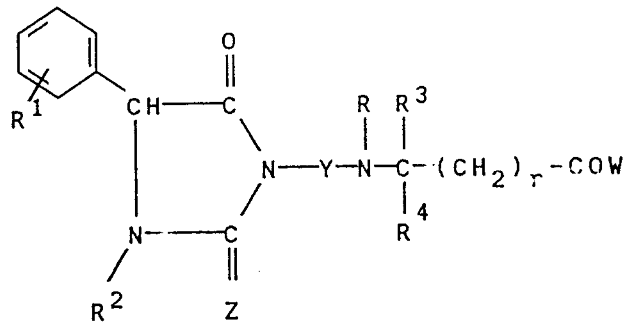

(I)

worin

Y -$(CH_2)_m$-CO-, wobei m für eine Zahl von 1 bis 4 steht, oder

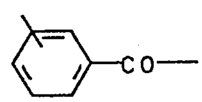

bedeutet;

r eine Zahl von 0 bis 3 bedeutet;

Z Sauerstoff oder Schwefel bedeutet;

W Hydroxy, ($C_1$-$C_{28}$)-Alkoxy, ($C_6$-$C_{14}$)-Aryl-($C_1$-$C_8$)-alkoxy, das im Arylrest auch substituiert sein kann, gegebenenfalls substituiertes ($C_6$-$C_{14}$)-Aryloxy, Amino oder Mono-oder Di-($C_1$-$C_{18}$)-Alkyl-amino bedeutet;

$R^1$ -$(CH_2)_n$-NH-X oder -$(CH_2)_p$-C(=NH)-NH-$X^1$, wobei n und p für eine Zahl 0 bis 3 stehen, bedeutet;

X,$X^1$ Wasserstoff, ($C_1$-$C_6$)-Alkyl, ($C_1$-$C_6$)-Alkoxycarbonyl, ($C_1$-$C_6$)-Alkylcarbonyl, ($C_1$-$C_{18}$)-Alkylcarbonyl-oxy ($C_1$-$C_6$)-alkoxycarbonyl, ($C_6$-$C_{14}$)-Aryloxycarbonyl, das im Arylrest auch substituiert sein kann, ($C_6$-$C_{14}$)-Aryl($C_1$-$C_6$)-alkoxycarbonyl, das im Arylrest auch substituiert sein kann, Cyano, Hydroxy, ($C_1$-$C_6$)-Alkoxy oder Amino bedeutet, und

X zusätzlich einen Rest der Formel II

$$R'\text{-NH-C(=N-}R'')\text{-} \qquad \text{(II)}$$

wobei

R', R" unabhängig voneinander Wasserstoff, ($C_1$-$C_6$)-Alkyl, ($C_1$-$C_6$)-Alkoxycarbonyl, ($C_1$-$C_6$)-Alkylcarbonyl, ($C_1$-$C_{18}$)-Alkylcarbonyl-oxy-($C_1$-$C_6$)-alkoxycarbonyl, ($C_6$-$C_{14}$)-Aryloxycarbonyl, das im Arylrest auch substituiert sein kann, ($C_6$-$C_{14}$)-Aryl($C_1$-$C_6$)-alkoxycarbonyl, das im Arylrest auch substituiert sein kann, Cyano, Hydroxy, ($C_1$-$C_6$)-Alkoxy oder Amino bedeutet;

R, $R^2$ Wasserstoff oder ($C_1$-$C_6$)-Alkyl bedeutet;

$R^3$ Wasserstoff, Phenyl oder substituiertes Phenyl bedeutet;

$R^4$ Wasserstoff, -$COOR^5$, -CO-N($CH_3$)$R^5$ oder -CO-NH-$R^5$ bedeutet;

$R^5$ Wasserstoff oder ($C_1$-$C_{28}$)-Alkyl bedeutet, das gegebenenfalls ein- oder mehrfach durch gleiche oder verschiedene Reste aus der Reihe Hydroxy, Hydroxycarbonyl, Aminocarbonyl, Mono- oder Di-($C_1$-$C_{18}$)-alkylaminocarbonyl, Amino-($C_2$-$C_{14}$)-alkylaminocarbonyl, Amino($C_1$-$C_3$)-alkylphenyl-($C_1$-$C_3$)-alkylaminocarbonyl, ($C_1$-$C_{18}$)-Alkylcarbonylamino-($C_1$-$C_3$)-alkylphenyl-($C_1$-$C_3$)-alkylaminocarbonyl, ($C_1$-

$C_{18}$)-Alkylcarbonylamino-($C_2$-$C_{14}$)-alkylaminocarbonyl, Phenyl-($C_1$-$C_8$)-alkoxycarbonyl, das im Arylrest auch substituiert sein kann, Amino, Mercapto, ($C_1$-$C_{18}$)-Alkoxy, ($C_1$-$C_{18}$)-Alkoxycarbonyl, gegebenenfalls substituiertes ($C_3$-$C_8$)-Cycloalkyl, Halogen, Nitro, Trifluormethyl oder einen Rest $R^6$ substituiert ist, wobei

$R^6$ gegebenenfalls substituiertes ($C_6$-$C_{14}$)-Aryl, gegebenenfalls substituiertes ($C_6$-$C_{14}$)-Aryl-($C_1$-$C_8$)-alkyl oder einen mono- oder bizyklischen 5- bis 12-gliedrigen heterozyklischen Ring, der aromatisch, teilhydriert oder vollständig hydriert sein kann und der als Heteroelement ein, zwei oder drei gleiche oder verschiedene Stickstoff-, Sauerstoff- oder Schwefelatome enthalten kann, oder einen Rest $R^7$ bedeutet wobei der Aryl- und unabhängig davon der Heterozyklus-Rest gegebenfalls ein- oder mehrfach durch gleiche oder verschiedene Reste aus der Reihe ($C_1$-$C_{18}$)-Alkyl, ($C_1$-$C_{18}$)-Alkoxy, Halogen, Nitro, Amino oder Trifluormethyl substituiert sein können;

$R^7$ -$NR^8R^9$, -$OR^8$, -$SR^8$, eine Aminosäureseitenkette, einen natürlichen oder unnatürlichen Aminosäure-, Iminosäure-, gegebenenfalls N-($C_1$-$C_8$)-alkylierten oder ($C_6$-$C_{14}$)-Aryl-($C_1$-$C_8$)-alkylierten Azaaminosäure- oder einen Dipeptid-Rest, der im Arylrest auch substituiert und/oder bei dem die Peptidbindung zu -NH-$CH_2$- reduziert sein kann, sowie deren Ester und Amide, wobei freie funktionelle Gruppen gegebenfalls durch Wasserstoff oder Hydroxymethyl substituiert oder durch in der Peptidchemie übliche Schutzgruppen geschützt sein können, oder einen Rest -$COR^{7'}$ bedeuten, worin $R^{7'}$ wie $R^7$ definiert ist, bedeutet;

$R^8$ Wasserstoff, ($C_2$-$C_{18}$)-Alkyl, gegebenenfalls substituiertes ($C_6$-$C_{14}$)-Aryl-($C_1$-$C_8$)alkyl, ($C_1$-$C_{18}$)-Alkylcarbonyl, ($C_1$-$C_{18}$)-Alkoxycarbonyl, ($C_6$-$C_{14}$)-Arylcarbonyl, ($C_6$-$C_{14}$)-Aryl-($C_1$-$C_8$)-alkylcarbonyl oder ($C_6$-$C_{14}$)-Aryl-($C_1$-$C_{18}$)-alkyloxycarbonyl, wobei die Alkylgruppen gegebenenfalls durch eine Aminogruppe und/oder wobei die Arylreste ein- oder mehrfach, vorzugsweise einfach durch gleiche oder verschiedene Reste aus der Reihe ($C_1$-$C_8$)Alkyl, ($C_1$-$C_8$)Alkoxy, Halogen, Nitro, Amino oder Trifluormethyl substituiert sein können, einen natürlichen oder unnatürlichen Aminosäure-, Iminosäure-, gegebenenfalls N-($C_1$-$C_8$)-alkylierten oder ($C_6$-$C_{14}$)-Aryl-($C_1$-$C_8$)-alkylierten Azaaminosäure- oder einen Dipeptid-Rest, der im Arylrest auch substituiert und/oder bei dem die Peptidbindung zu -NH-$CH_2$- reduziert sein kann, bedeutet; und

$R^9$ Wasserstoff, ($C_1$-$C_{18}$)-Alkyl, gegebenenfalls substituiertes ($C_6$-$C_{14}$)-Aryl oder ($C_6$-$C_{14}$)-Aryl-($C_1$-$C_8$)-alkyl, das im Arylrest auch substituiert sein kann, bedeutet;

sowie deren physiologisch verträgliche Salze.

Alkylreste können geradkettig oder verzweigt sein. Bevorzugte Alkylreste sind Methyl, Ethyl, n-Propyl, i-Propyl, n-Butyl, i-Butyl, sek-Butyl und tert.-Butyl. Entsprechendes gilt für Reste wie Alkoxy, Alkoxycarbonyl oder Aralkyl. ($C_3$-$C_8$)-Cycloalkylreste sind insbesondere Cyclopropyl, Cyclobutyl, Cyclopentyl, Cyclohexyl, Cycloheptyl und Cyclooctyl, die aber auch durch beispielsweise ($C_1$-$C_4$)-Alkyl substituiert sein können. Beispiele für substituierte Cycloalkylreste sind 4-Methylcyclohexyl und 2,3-Dimethylcyclopentyl.

($C_6$-$C_{14}$)-Arylgruppen sind beispielsweise Phenyl, Naphthyl, Biphenylyl oder Fluorenyl, wobei Phenyl und Naphthyl bevorzugt sind. Entsprechendes gilt für Reste wie Aralkyl oder Arylcarbonyl. Aralkylreste sind insbesondere Benzyl sowie 1- und 2-Naphthylmethyl, die auch substituiert sein können. Die Arylreste, insbesondere Phenylreste, können, auch wenn sie als Substituenten anderer Reste auftreten, auch ein- oder mehrfach durch gleiche oder verschiedene Reste aus der Reihe ($C_1$-$C_8$)-Alkyl, ($C_1$-$C_8$)-Alkoxy, Halogen, Nitro, Amino oder Trifluormethyl substituiert sein. Substituierte Aralkylreste sind beispielsweise Halobenzyl oder ($C_1$-$C_4$)-Alkoxybenzyl.

Ist Phenyl zweifach substituiert, können die Substituenten in 1,2-, 1,3- oder 1,4-Position zueinander stehen. Bei einer Disubstitution ist die 1,3- sowie die 1,4-Position bevorzugt.

Heterocyclen im Sinne vorstehender Definitionen sind beispielsweise Pyrrolyl, Furyl, Thienyl, Imidazolyl, Pyrazolyl, Oxazolyl, Isoxazolyl, Thiazolyl, Isothiazolyl, Tetrazolyl, Pyridyl, Pyrazinyl, Pyrimidinyl, Indolyl, Isoindazolyl, Indazolyl, Phthalazinyl, Chinolyl, Isochinolyl, Chinoxalinyl, Chinazolinyl, Cinnolinyl oder ein benzanelliertes, cyclopenta-, cyclohexa- oder cyclohepta-anelliertes Derivat dieser Reste.

Diese Heterocyclen können an einem Stickstoffatom durch Oxide, ($C_1$-$C_7$)-Alkyl, z. B. Methyl oder Ethyl, Phenyl oder Phenyl-($C_1$-$C_4$)-alkyl, z. B. Benzyl und/oder an einem oder mehreren Kohlenstoffatomen durch ($C_1$-$C_4$)-Alkyl, Halogen, Hydroxy, ($C_1$-$C_4$)-Alkoxy, z. B. Methoxy, Phenyl-($C_1$-$C_4$)-alkoxy, z. B. Benzyloxy oder Oxo substituiert und teilweise oder vollständig gesättigt sein.

Derartige Reste sind beispielsweise 2- oder 3-Pyrrolyl, Phenyl-pyrrolyl, z. B. 4-oder 5-Phenyl-2-pyrrolyl, 2-Furyl, 2-Thienyl, 4-Imidazolyl, Methyl-imidazolyl, z.B. 1-Methyl-2-, -4- oder -5-imidazolyl, 1,3-Thiazol-2-yl, -2-, -3- oder -4-pyridyl, 2-, 3- oder 4-Pyridyl-N-oxid, 2-Pyrazinyl, 2-, 4- oder 5-Pyrimidinyl, 2-, 3- oder 5-Indolyl, substituiertes 2-Indolyl, z. B. 1-Methyl-, 5-Methyl-, 5-Methoxy-, 5-Benzyloxy-, 5-Chlor- oder 4,5-Dimethyl-2-indolyl, 1-Benzyl-2- oder -3-indolyl, 4,5,6,7-Tetrahydro-2-indolyl, Cyclohepta[b]-5-pyrrolyl, 2-, 3- oder 4-Chinolyl, 1-, 3- oder 4-Isochinolyl, 1-Oxo-1,2-dihydro-3-isochinolyl, 2-Chinoxalinyl, 2-Benzofuranyl, 2-Benzothienyl, 2-Benzoxazolyl oder Benzothiazolyl. Teilhydrierte oder vollständig hydrierte heterocyclische Ringe sind beispielsweise Dihydropyridinyl, Pyrrolidinyl, z. B. 2-, 3- oder 4-N-methylpyrrolidinyl, Piperazinyl, Morpholinyl, Thiomorpholinyl, Tetrahydrothienyl, Benzodioxolanyl.

Halogen steht für Fluor, Chlor, Brom oder Jod, insbesondere für Fluor oder Chlor.

Natürliche und unnatürliche Aminosäuren können, falls chiral, in der D- oder L-Form vorliegen. Bevorzugt sind α-Aminosäuren. Beispielsweise seien genannt (vgl. Houben-Weyl, Methoden der organischen Chemie, Band XV/1 und 2, Stuttgart, 1974):

Aad, Abu cAbu, ABz, 2ABz, ∊Aca, Ach, Acp, Adpd, Ahb, Aib, βAib, Ala, βAla, DAla, Alg, All, Ama, Amt, Ape, Apm, Apr, Arg, Asn, Asp, Asu, Aze, Azi, Bai, Bph, Can, Cit, Cys, $(Cys)_2$, Cyta, Daad, Dab, Dadd, Dap, Dapm, Dasu, Djen, Dpa, Dtc, Fel, Gln, Glu, Gly, Guv, hAla, hArg, hCys, hGln, hGlu, His, hIle, hLeu, hLys, hMet, hPhe, hPro, hSer, hThr, hTrp, hTyr, Hyl, Hyp, 3Hyp, Ile, Ise, Iva, Kyn, Lant, Lcn, Leu, Lsg, Lys, βLys, DLys, Met, Mim, Min, nArg, Nle, Nva, Oly, Orn, Pan, Pec, Pen, Phe, Phg, Pic, Pro, DPro, Pse, Pya, Pyr, Pza, Qin, Ros, Sar, Sec, Sem, Ser, Thi, βThi, Thr, Thy, Thx, Tia, Tle, Tly, Trp, Trta, Tyr, Val, Tbg, Npg, Chg, Cha, Thia, 2,2-Diphenylaminoessigsäure, 2-(p-Tolyl)2-phenylaminoessigsäure, 2-(p-Chlorphenyl)aminoessigsäure.

Unter Aminosäureseitenketten werden Seitenketten von natürlichen oder unnatürlichen Aminosäuren verstanden. Azaaminosäuren sind natürliche oder unnatürliche Aminosäuren, wobei der Zentralbaustein -CHR- bzw. $-CH_2-$durch -NR- bzw. -NH- ersetzt ist.

Als Rest einer Iminosäure kommen insbesondere Reste von Heterocyclen aus der folgenden Gruppe in Betracht: Pyrrolidin-2-carbonsäure; Piperidin-2-carbonsäure; Tetrahydroisochinolin-3-carbonsäure; Decahydroisochinolin-3-carbonsäure; Octahydroindol-2-carbonsäure; Decahydrochinolin-2-carbonsäure; Octahydrocyclopenta[b]pyrrol-2-carbonsäure; 2-Aza-bicyclo-[2.2.2]octan-3-carbonsäure; 2-Azabicyclo[2.2.1]heptan-3-carbonsäure; 2-Azabicyclo[3.1.0]hexan-3-carbonsäure; 2-Azaspiro[4.4]nonan-3-carbonsäure; 2-Azaspiro[4.5]decan-3-carbonsäure; Spiro(bicyclo[2.2.1]-heptan)-2,3-pyrrolidin-5-carbonsäure; Spiro(bicyclo[2.2.2]octan)-2,3-pyrrolidin-5-carbo nsäure; 2-Azatricyclo[4.3.0.1$^{6,9}$]decan-3-carbonsäure; Decahydrocyclohepta[b]pyrrol-2-carbonsäure; Decahydrocycloocta[c]pyrrol-2-carbonsäure; Octahydrocyclopenta[c]pyrrol-2-carbonsäure; Octahydroisoindol-1-carbonsäure; 2,3,3a,4,6a-Hexahydrocyclopenta[b]pyrrol-2-carbonsäure; 2,3,3a,4,5,7a-Hexahydroindol-2-carbonsäure; Tetrahydrothiazol-4-carbonsäure; Isoxazolidin-3-carbonsäure; Pyrazolidin-3-carbonsäure; Hydroxyprolin-2-carbonsäure; die alle gegebenenfalls substituiert sein können (siehe folgende Formeln):

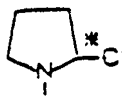 ; 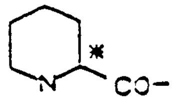 ;

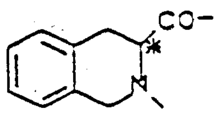 ; 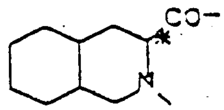 ; 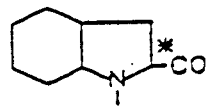 ; 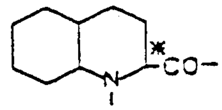 ;

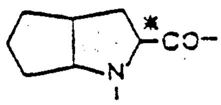 ; 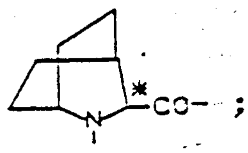 ; 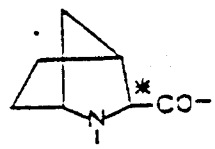 ; 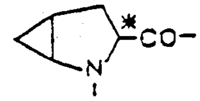 ;

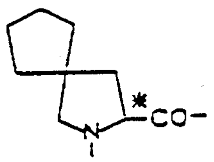 ; 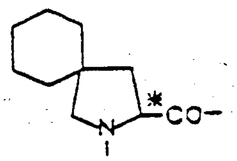 ; 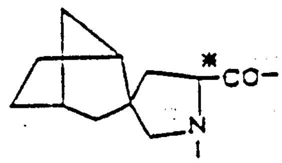 ;

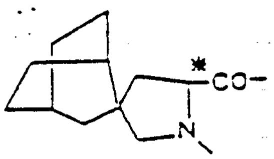 ; 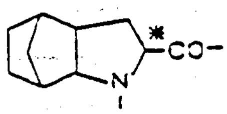 ; 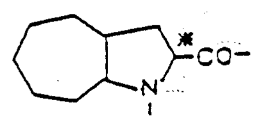 ;

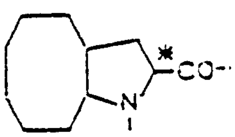 ; 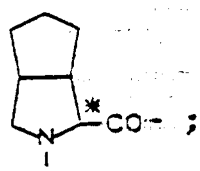 ; 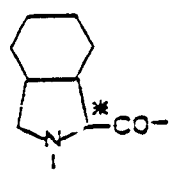 ;

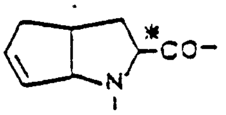 ; 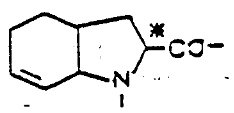 ; 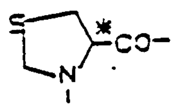 ;

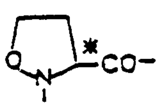 ; 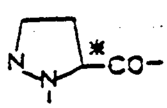 ; 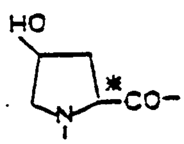 ;

Die oben genannten Resten zugrundeliegenden Heterocyclen sind beispielsweise bekannt aus US-A 4,344,949; US-A 4,374,847; US-A 4,350,704; EP-A 29,488; EP-A 31,741; EP-A 46,953; EP-A 49,605; EP-A 49,658; EP-A 50,800; EP-A 51,020; EP-A 52,870; EP-A 79,022; EP-A 84,164; EP-A 89,637; EP-A 90,341; EP-A 90,362; EP-A 105,102; EP-A 109,020; EP-A 111,873; EP-A 271,865 und EP-A 344,682.

Dipeptide könne als Bausteine natürliche oder unnatürliche Aminosäuren, Iminosäuren sowie Azaaminosäuren enthalten. Ferner können die natürlichen oder unnatürlichen Aminosäuren, Iminosäuren, Azaaminosäuren und Dipeptide auch als Ester bzw. Amide vorliegen, wie z. B. Methylester, Ethylamid, Semicarbazid oder x-Amino-($C_4$-$C_8$)-alkylamid.

Funktionelle Gruppen der Aminosäuren, Iminosäuren und Dipeptide können geschützt vorliegen. Geeignete Schutzgruppen wie z. B. Urethanschutzgruppen, Carboxylschutzgruppen und Seitenkettenschutzgruppen sind bei Hubbuch, Kontakte (Merck) 1979, Nr. 3, Seiten 14 bis 23 und bei Büllesbach, Kontakte (Merck) 1980, Nr. 1, Seiten 23 bis 35 beschrieben. Insbesondere seien genannt: Aloc, Pyoc, Fmoc, Tcboc, Z, Boc, Ddz, Bpoc, Adoc, Msc, Moc, $Z(NO_2)$, $Z(Hal_n)$, Bobz, Iboc, Adpoc, Mboc, Acm, tert.-Butyl, OBzl, ONbzl, OMbzl, Bzl, Mob, Pic, Trt.

Physiologisch verträgliche Salze der Verbindungen der allgemeinen Formel I sind insbesondere pharmazeutisch verwendbare oder nicht-toxische Salze.

Solche Salze werden beispielsweise von Verbindungen der allgemeinen Formel I, welche saure Gruppen, z. B. Carboxy, enthalten, mit Alkali- oder Erdalkalimetallen gebildet, wie z. B. Na, K, Mg und Ca, sowie mit physiologisch verträglichen organischen Aminen, wie z. B. Triethylamin und Tris-(2-hydroxy-ethyl)-amin.

Verbindungen der allgemeinen Formel I, welche basische Gruppen, z. B. eine Aminogruppe oder eine Guanidinogruppe enthalten, bilden mit anorganischen Säuren, wie z. B. Salzsäure, Schwefelsäure oder Phosphorsäure und mit organischen Carbon-oder Sulfonsäuren, wie z. B. Essigsäure, Citronensäure, Benzoesäure, Maleinsäure, Fumarsäure, Weinsäure und p-Toluolsulfonsäure Salze.

Bevorzugte Verbindungen der allgemeinen Formel I sind solche, worin

Y $-(CH_2)_m-CO-$, wobei m für 1 oder 2 steht, oder

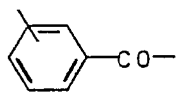

bedeutet;
r 1 bedeutet;
Z Sauerstoff oder Schwefel bedeutet;
W Hydroxy, $(C_1-C_6)$-Alkoxy, besonders Methoxy, Ethoxy oder 2-Propyloxy bedeutet;
R Wasserstoff bedeutet;
$R^1$ $-NH-C(=NH)-NH_2$, $-C(=NH)-NH_2$ oder $-CH_2-NH_2$ oder Methoxycarbonylderivate davon bedeutet;
$R^2$ Wasserstoff oder Methyl bedeutet;
$R^3$ Wasserstoff bedeutet; und
$R^4$ $-CO-NH-R^5$ bedeutet, wobei $-NH-R^5$ für einen $\alpha$-Aminosäurerest oder dessen x-Amino-$(C_2-C_8)$-alkylamid steht.

Für $-NH-R^5$ stehende $\alpha$-Aminosäurereste sind dabei besonders bevorzugt der Valin-, Lysin-, Phenylalanin- oder Phenylglycin-Rest. Ein besonders bevorzugtes x-Amino-$(C_2-C_8)$-alkylamid ist das 4-Aminobutylamid.

Die erfindungsgemäßen Verbindungen der Formel I können hergestellt werden durch Fragmentkondensation einer Verbindung der allgemeinen Formel III

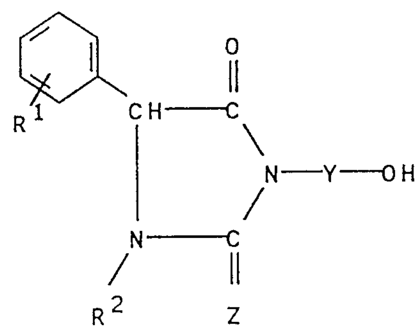

(III)

mit einer Verbindung der allgemeinen Formel IV

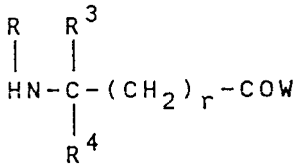

(IV)

wobei r und die Reste R, $R^1$ bis $R^4$, Y, Z und W wie oben angegeben definiert sind.

Die Ausgangspeptide der Formel IV werden in der Regel vom C-terminalen Ende her stufenweise aufgebaut. Zur Kondensation der Verbindungen der allgemeinen Formel III mit denen der allgemeinen Formel IV verwendet man vorteilhafterweise die an sich bekannten Kupplungsmethoden der Peptidchemie (s. z. B. Houben Weyl, Methoden der Organischen Chemie, Band 15/1 und 15/2, Stuttgart, 1974). Dazu ist es in der Regel nötig, daß in $R^1$ und $R^4$ enthaltene Aminogruppen durch reversible Schutzgruppen während der Kondensation geschützt werden. Gleiches gilt für die Carboxylgruppen der Verbindungen der Formel IV, die bevorzugt als ($C_1$-$C_6$)-Alkyl, Benzyl- oder tert.-Butylester vorliegen. Ein Aminogruppen-Schutz erübrigt sich, wenn die zu generierenden Aminogruppen noch als Nitro- oder Cyanogruppen vorliegen und erst nach der Kupplung durch Hydrierung gebildet werden. Nach der Kupplung werden die vorhandenen Schutzgruppen in geeigneter Weise abgespalten. Beispielsweise können $NO_2$-Gruppen (Guanidinoschutz), Benzyloxycarbonylgruppen und Benzylester abhydriert werden. Die Schutzgruppen vom tert.-Butyltyp werden sauer gespalten, während der 9-Fluorenylmethyloxycarbonylrest durch sekundäre Amine entfernt wird.

Die Ausgangsverbindungen der allgemeinen Formel III können wie folgt erhalten werden:

Durch Reaktion von Aminosäuren, N-Alkylaminosäuren oder bevorzugt deren Ester (z.B. Methyl-, Ethyl-, Benzyl- oder tert.-Butylester), beispielsweise einer Verbindung der allgemeinen Formel V

$$R^2\text{-NH-CH-}(C_6H_4\text{-}R^1)\text{-COOCH}_3 \qquad (V)$$

mit einem Isocyanatoalkancarbonsäureester, einem Isothiocyanatoalkancarbonsäureester oder einem Isocyanat oder Isothiocyanat der Aminobenzoesäure, beispielsweise der allgemeinen Formel VI

$$Z{=}C{=}N\text{-}Y\text{-COOCH}_3 \qquad (VI)$$

worin $R^1$, $R^2$, Y und Z wie oben angegeben definiert sind, erhält man Harnstoff- oder Thioharnstoffderivate, beispielsweise der allgemeinen Formel VII

$$CH_3OOC\text{-}Y\text{-NH-CZ-N}(R^2)\text{-CH}(C_6H_4\text{-}R^1)\text{-COOCH}_3 \qquad (VII)$$

die durch Erhitzen mit Säure unter Verseifung der Esterfunktionen zu Verbindungen der allgemeinen Formel III zyklisieren. Während der Zyklisierung können Guanidinogruppen durch Schutzgruppen (z.B. $NO_2$ oder Mtr) blockiert werden. Aminogruppen in der Seitenkette können in geschützter Form (z.B. als Boc oder Z-Derivate) oder noch als $NO_2$-oder Cyanofunktion vorliegen, die später zur Aminogruppe reduziert oder im Falle der Cyanogruppe auch in die Formamidinogruppe umgewandelt werden kann.

Im übrigen entstehen Hydantoine der allgemeinen Formel VIII

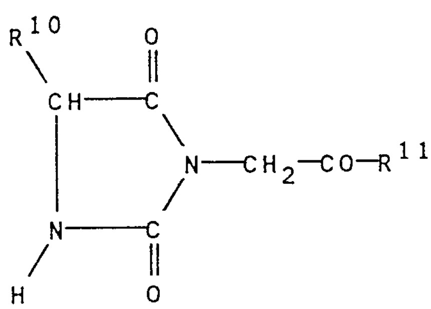

(VIII)

worin $R^{10}$ eine beliebige Aminosäureseitenkette und $R^{11}$ ein Amid, einen Aminosäure- oder einen Peptidrest bedeuten, ganz allgemein durch basische Behandlung von Alkyloxycarbonyl- oder Aralkyloxycarbonyl-Peptiden der allgemeinen

Formel IX

$$R^{12}\text{-O-CO-NH-CHR}^{10}\text{-CO-NH-CH}_2\text{-CO-R}^{11} \qquad \text{(IX)}$$

worin $R^{10}$ und $R^{11}$ wie oben angegeben definiert sind und $R^{12}$ Benzyl- oder tert.-Butyl bedeutet ( J.S. Fruton und M. Bergmann, J. Biol. Chem. 145 (1942) 253-265; C.A. Dekker, S.P. Taylor,jr. und J.S. Fruton, J. Biol. Chem. 180 (1949) 155-173; M.E. Cox, H.G. Garg, J. Hollowood, J.M. Hugo, P.M. Scopes und G.T. Young, J. Chem. Soc. (1965) 6806-6813; W. Voelter und A. Altenburg, Liebigs Ann. Chem. (1983) 1641-1655; B. Schwenzer, E. Weber und G. Losse, J. Prakt. Chem. 327 (1985) 479-486 ). Dabei razemisiert jedoch die N-terminale Aminosäure und das Hydantoin hydrolysiert in das Harnstoffderivat

$$\text{HOCO-CHR}^{10}\text{-NH-CO-NH-CH}_2\text{-CO-R}^{11}$$

(W. Voelter und A. Altenburg, Liebigs Ann.Chem. (1983) 1641-1655).

Eine milde Methode ist dagegen die Zyklisierung zu den Hydantoinen aus Verbindungen der Formel IX durch Behandlung mit Tetrabutylammoniumfluorid in Tetrahydrofuran unter Rückfluß (J. Pless, J. Org. Chem. 39 (1974) 2644 - 2646).

Eine weitere Möglichkeit einer milden Zyklisierung ist die Trimethylsilylierung der Peptidbindung zwischen der N-terminalen Aminosäure und dem folgenden Glycin mit Bistrimethylsilyltrifluoracetamid in Acetonitril (4 Stunden unter Rückfluß) ( J.S. Davies, R. K. Merritt und R. C. Treadgold, J. Chem. Soc. Perkin Trans. I (1982) 2939 - 2947).

Die Guanylierung der Aminofunktion kann mit folgenden Reagentien durchgeführt werden:

1. O-Methylisoharnstoff (S.Weiss und H. Krommer, Chemiker Zeitung 98 (1974) 617-618),
2. S-Methylisothioharnstoff (R.F. Borne, M.L.Forrester und I.W. Waters, J. Med. Chem. 20 (1977) 771-776)
3. Nitro-S-Methylisothioharnstoff (L.S. Hafner und R.E. Evans, J. Org. Chem. 24 (1959) 1157),
4. Formamidinosulfonsäure (K. Kim, Y.-T. Lin und H.S. Mosher, Tetrah. Lett. 29 (1988) 3183-3186)
5. 3,5-Dimethyl-1-pyrazolyl-formamidinium-nitrat (F.L. Scott, D.G. O'Donovan und J. Reilly, J. Amer.Chem. Soc. 75 (1953) 4053-4054).
6. N,N'-Di-tert.-butyloxycarbonyl-S-methyl-isothioharnstoff (R.J.Bergeron und J.S.McManis, J.Org.Chem. 52 (1987), 1700-1703).

Formamidine können aus den entsprechenden Cyanoverbindungen durch Anlagerung von Alkoholen (z. B. Methanol oder Ethanol) in saurem wasserfreiem Medium (z. B. Dioxan, Methanol oder Ethanol) und anschließender Behandlung mit Ammoniak in Alkoholen (z. B. Isopropanol, Methanol oder Ethanol) hergestellt werden (G. Wagner, P. Richter und Ch. Garbe, Pharmazie 29 (1974) 12-55). Eine weitere Methode, Formamidine herzustellen, ist die Anlagerung von $H_2S$ an die Cyanogruppe, gefolgt von einer Methylierung des entstandenen Thioamids und anschließender Umsetzung mit Ammoniak (DDR-Patent Nr. 235 866).

Eine weitere Methode zur Herstellung der Verbindungen der allgemeinen Formel III ist die Umsetzung von Verbindungen der Formel X

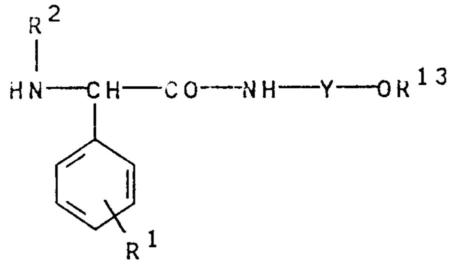

(X)

wobei $R^{13}$ Wasserstoff oder ($C_1$-$C_6$)-Alkyl bedeutet, mit Phosgen, Thiophosgen oder entsprechenden Äquivalenten zu den Estern der Imidazolidinderivate, die anschließend zu den Carbonsäuren verseift werden können (analog S. Goldschmidt und M. Wick, Liebigs Ann.Chem. 575 (1952) 217-231, C. Tropp, Chem. Ber. 61, (1928) 1431-1439).

Die Verbindungen der allgemeinen Formel I und ihre physiologisch verträglichen Salze können als Heilmittel für sich allein, in Mischungen untereinander oder in Form von pharmazeutischen Zubereitungen verabreicht werden, die eine enterale oder parenterale Anwendung gestatten und die als aktiven Bestandteil eine wirksame Dosis mindestens einer Verbindung der allgemeinen Formel I oder eines Salzes davon, neben üblichen pharmazeutisch einwandfreien Träger- und Zusatzstoffen enthalten. Die Zubereitungen enthalten normalerweise etwa 0,5 bis 90 Gew.% der therapeutisch wirksamen Verbindung.

Die Heilmittelm können oral, z.B. in Form von Pillen, Tabletten, Lacktabletten, Dragees, Granulaten, Hart- und Weichgelatinekapseln, Lösungen, Sirupen, Emulsionen oder Suspensionen oder Aerosolmischungen verabreicht werden. Die Verabreichung kann aber auch rektal, z.B. in Form von Suppositorien oder parenteral, z.B. in Form von Injektionslösungen, Mikrokapseln oder Rods, perkutan, z.B. in Form von Salben oder Tinkturen, oder nasal, z.B. in Form von Nasalsprays, erfolgen.

Die Herstellung der pharmazeutischen Präparate erfolgt in an sich bekannter Weise, wobei pharmazeutisch inerte anorganische oder organische Trägerstoffe verwendet werden. Für die Herstellung von Pillen, Tabletten, Dragees und Hartgelatinekapseln kann man z.B. Lactose, Maisstärke oder Derivate davon, Talk, Stearinsäure oder deren Salze etc. verwenden. Trägerstoffe für Weichgelatinekapseln und Suppositorien sind z.B. Fette, Wachse, halbfeste und flüssige Polyole, natürliche oder gehärtete Öle etc. Als Trägerstoffe für die Herstellung von Lösungen und Sirupen eignen sich z.B. Wasser, Saccharose, Invertzucker, Glukose, Polyole etc. Als Trägerstoffe für die Herstellung von Injektionslösungen eignen sich Wasser, Alkohole, Glycerin, Polyole, pflanzliche Öle etc. Als Trägerstoffe für Mikrokapseln, Implantate oder Rods eignen sich Mischpolymerisate aus Glykolsäure und Milchsäure.

Die pharmazeutischen Präparate können neben den Wirk- und Trägerstoffen noch Zusatzstoffe, wie z.B. Füllstoffe, Streck-, Spreng-, Binde-, Gleit-, Netz-, Stabilisierungs-, Emulgier-, Konservierungs-, Süß-, Färbe-, Geschmacks- oder Aromatisierungs-, Dickungs-, Verdünnungmittel, Puffersubstanzen, ferner Lösungsmittel oder Lösungsvermittler oder Mittel zur Erzielung eines Depoteffekts, sowie Salze zur Veränderung des osmotischen Drucks, Überzugsmittel oder Antioxidantien enthalten. Sie können auch zwei oder mehrere Verbindungen der allgemeinen Formel I oder ihrer pharmakologisch annehmbaren Säureadditionssalze und noch einen oder mehrere andere therapeutisch wirksame Stoffe enthalten.

Derartige andere therapeutisch wirksame Substanzen sind beispielsweise durchblutungsfördernde Mittel, wie Dihydroergocristin, Nicergolin, Buphenin, Nicotinsäure und ihre Ester, Pyridylcarbinol, Bencyclan, Cinnarizin, Naftidrofuryl, Raubasin und Vincamin; positiv inotrope Verbindungen, wie Digoxin, Acetyldigoxin, Metildigoxin und Lantano-Glykoside; Coronardilatatoren, wie Carbocromen; Dipyridamol, Nifedipin und Perhexilin; antianginöse Verbindungen, wie Isosorbiddinitrat, Isosorbidmononitrat, Glycerolnitrat, Molsidomin und Verapamil; β-Blocker, wie Propranolol, Oxprenolol, Atenolol, Metoprolol und Penbutolol. Darüberhinaus lassen sich die Verbindungen mit anderen nootrop wirksamen Substanzen, wie z.B. Piracetam, oder ZNS-aktiven Substanzen, wie Pirlindol, Sulpirid etc. kombinieren.

Die Dosis kann innerhalb weiter Grenzen variieren und ist in jedem einzelnen Fall den individuellen Gegebenheiten anzupassen. Im allgemeinen ist bei der oralen Verabreichung eine Tagesdosis von etwa 0,1 bis 1 mg/kg, vorzugsweise 0,3 bis 0,5 mg/kg Körpergewicht zur Erzielung wirksamer Ergebnisse angemessen, bei intravenöser Applikation beträgt die Tagesdosis im allgemeinen etwa 0,01 bis 0,3 mg/kg, vorzugsweise 0,05 bis 0,1 mg/kg Körpergewicht. Die Tagesdosis wird normalerweise, insbesondere bei der Applikation größerer Mengen, in mehrere, z.B. 2, 3, oder 4 Teilverabreichungen aufgeteilt. Gegebenenfalls kann es, je nach individuellem Verhalten, erforderlich werden, von der angegebenen Tagesdosis nach oben oder nach unten abzuweichen. Pharmazeutische Präparate enthalten normalerweise 0,2 bis 50 mg, vorzugsweise 0,5 bis 10 mg Wirkstoff der allgemeinen Formel I oder eines ihrer physiologisch verträglichen Salze pro Dosis.

Die erfindungsgemäßen Verbindungen der Formel I haben die Fähigkeit die Zell-Zell-Adhäsion zu hemmen, die auf der Interaktion von Arg-Gly-Asp-enthaltenden Proteinen, wie Fibronectin, Fibrinogen oder des von Willebrand-Faktors mit den sogenannten Integrinen beruhen. Integrine sind Transmembran-Glykoproteine, Rezeptoren für Arg-Gly-Asp-enthaltende Zellmatrix-Glykoproteine, (E. Ruoslahti und M.D. Pierschbacher, Science 238 (1987) 491-497; D.R. Phillips, I.F. Charo, L.V. Parise und L.A. Fitzgerald, Blood 71 (1988) 831-843). Außerdem hemmen sie die Bindung weiterer adhäsiver Proteine, wie Vitronectin, Kollagen und Laminin an die entsprechenden Rezeptoren auf der Oberfläche verschiedener Zelltypen.

Die erfindungsgemäßen Verbindungen der Formel I hemmen die Thrombozytenaggregation, die Metastasierung von Karzinomzellen sowie die Osteoclastenbindung an die Knochenoberflächen.

Die erfindungsgemäßen Verbindungen der Formel I finden akut Anwendung bei Thrombosegefahr und chronisch bei der Prävention der Arteriosklerose und Thrombose, z.B. bei der Therapie und Prophylaxe arterieller Gefäßerkrankungen, wie bei akutem Myokardinfarkt, Sekundärprävention des Myokardinfarkts, Reokklusionsprophylaxe nach Lyse und Dilatation (PTCA), instabiler Angina pectoris, transitorischen ischämischen Attacken, Schlaganfall, koronarer Bypass-Operation einschließlich Reokklusionsprophylaxe bei Bypass, Lungenembolie, peripherer arterieller Verschlußkrankheit, Dissezierendem Aneurysma; bei der Therapie venöser und mikrozirkulatorischer Gefäßerkrankungen, wie tiefer Venenthrombose, disseminenter intravaskulärer Gerinnung, postoperativem und post-partum Trauma, chirurgischem oder infektiösem Schock, Septicämie oder bei Erkrankungen mit hyperreagiblen Thrombozyten, thrombotischer thrombozytopenischer Purpura, Preeklampsie, prämenstruellem Syndrom, Dialyse oder extrakorporaler Zirkulation; eine weitere Anwendung ist während Krebsoperationen und auch prophylaktisch bei Krebs gegeben. Ferner kann Osteoporose durch Hemmung der Osteoclastenbindung an die Knochenoberfläche verhindert werden.

Geprüft werden die Verbindungen vor allem auf ihre hemmende Wirkung bei der Blutplättchenaggregation und der

Anhaftung von Fibrinogen an Blutplättchen. Verwendet werden gelfiltrierte Blutplättchen aus humanem Spenderblut, die mit ADP oder Thrombin aktiviert werden.

Geprüft wird die Hemmung der Bindung von Fibrinogen an seinen Rezeptor (Glykoprotein IIb/IIIa) an intakten, gelfiltrierten Human-Thrombozyten durch die erfindungsgemäßen Verbindungen. Angegeben ist der Ki-Wert der Bindungshemmung von $^{125}$I-Fibrinogen nach Stimulierung mit ADP (10 μM). (Literatur: J.S. Bennett u. G. Vilaire, J. Clin. Invest. 64 (1979), 1393-1401; E. Kornecki et al., J. Biol. Chem. 256 (1981), 5695-5701; G.A. Marguerie et al., J. Biol. Chem. 254 (1979), 5357-5363; G.A. Marguerie et al., J. Biol. Chem. 255 (1980), 154-161).

Bei dieser Prüfung werden für die Verbindungen der nachfolgenden Beispiele 1 und 2 folgende Ergebnisse erhalten:

| Beispiel | Ki (μM), ADP stimuliert |
|---|---|
| 1 | 0,03 |
| 2 | 2 |

Als funktioneller Test wird die Hemmung der Aggregation gelfiltrierter Human-Thrombozyten nach ADP-oder Thrombin-Stimulierung durch die erfindungsgemäßen Verbindungen gemessen. Angegeben ist der $IC_{50}$-Wert der Hemmung. (Literatur: G.A. Marguerie et al., J. Biol. Chem. 254 (1979), 5357-5363)

Bei dieser Prüfung wurden für die Verbindungen der nachstehenden Beispiele 1 und 2 folgende Ergebnisse erhalten:

| Beispiel | ADP-stimuliert | Thrombin-stimuliert |
|---|---|---|
| | $IC_{50}$(μM) | $IC_{50}$(μM) |
| 1 | 0,2 | 0,07 |
| 2 | 6 | 3 |

Beispiele:

Die Produkte wurden über Massenspektren und/oder NMR-Spektren identifiziert.

Beispiel 1:

**(5-(4-Guanidinophenyl)-2,4-dioxoimidazolidin-3-yl)-acetyl-L-aspartyl-L-phenylglycin**

1a:

**N-(1-Methoxycarbonyl-(4-aminophenyl)-methyl),N'-ethoxycar bonylmethyl-harnstoff**

870 mg (4 mmol) 4-Amino-phenylglycinmethylester-dihydrochlorid werden in 10 ml Dimethylformamid gelöst. Nach Zugabe von 1 ml (8 mmol) N-Ethylmorpholin werden langsam 520 mg (4 mmol) Isocyanatoessigsäureethylester bei -20°C zugetropft. Man läßt auf Raumtemperatur erwärmen und rührt 15 Stunden bei Raumtemperatur, engt ein, löst in Essigester und extrahiert mit einer verdünnten Kaliumhydrogensulfatlösung. Nach dem Trocknen wird die organische Lösung eingeengt.

Ausbeute: 1.2 g

1b:

**(5-(4-Aminophenyl)-2,4-dioxo-imidazolidin-3-yl)-essigsäure**

1.2 g (3.9 mmol) N-(1-Methoxycarbonyl-(4-aminophenyl)-methyl), N'-ethoxycarbonylmethyl-harnstoff werden in 20

ml 6 N Salzsäure 30 Minuten unter Rückfluß erhitzt und im Vakuum eingeengt.

Ausbeute: 1.0 g (92%)

1c:

**(5-(4-Nitroguanidinophenyl)-2,4-dioxo-imidazolidin-3-yl)-essigsäure**

680 mg (5 mmol) Nitro-S-Methylisothioharnstoff und 1 g (3.5 mmol) (5-(4-Aminophenyl)-2,4-dioxo-imidazolidin-3-yl)-essigsäure werden in 37 ml 0.1 molarer Natronlauge 7 h bei 80 °C gerührt. Nach dem Abkühlen wird mit Methylenchlorid extrahiert, die Wasserphase klarfiltriert und mit verdünnter Salzsäure auf pH 3 angesäuert. Nach dem Einengen wird zur Reinigung an Sephadex LH20 mit einer homogenen Mischung von Butanol/Eisessig/Wasser chromatographiert.

Ausbeute: 460 mg

1d:

**(5-(4-Nitroguanidinophenyl)-2,4-dioxo-imidazolidin-3-yl)-acetyl-L-aspartyl(OtBu)-L-phenylglycin-OtBu**

Zu einer Lösung von 160 mg (0.475 mmol) (5-(4-Nitroguanidinophenyl)-2,4-dioxo-imidazolidin-3-yl)-essigsäure, 204 mg (0.475 mmol) H-Asp(OtBu)-phenylglycin-OtBu-hydrochlorid, 65 mg (0.48 mmol) Hydroxybenzotriazol in 10 ml Dimethylformamid gibt man bei 0°C 55 mg (0.477 mmol) N-Ethylmorpholin und 108 mg (0.523 mmol) DCC. Man rührt 1 Stunde bei 0°C und anschließend 5 Stunden bei Raumtemperatur. Der ausgefallene Harnstoff wird abgesaugt, das Filtrat eingeengt und das Rohprodukt über eine Kieselgelsäule mit Essigester/Methanol = 95:5 chromatographiert.

Ausbeute: 304 mg (92%)

1e:

**(5-(4-Guanidinophenyl)-2,4-dioxoimidazolidin-3-yl)-acetyl-L-aspartyl-L-phenylglycin**

Man läßt 300 mg (0.43 mmol) (5-(4-Nitroguanidinophenyl)-2,4-dioxo-imidazolidin-3-yl)-acetyl-L-aspartyl(OtBu)-L-phenylglycin-OtBu in 10 ml 95 proz. Trifluoressigsäure unter gelegentlichem Umschütteln 3 Stunden bei Raumtemperatur stehen und engt ein. Der Rückstand wird in 50 ml Methanol gelöst und nach Zugabe von 50 mg 10% Pd auf Kohle 5 h bei Raumtemperatur hydriert. Der Katalysator wird abfiltriert, das Filtrat eingeengt und der Rückstand zur Reinigung an Sephadex LH20 mit einer homogenen Mischung von Butanol/Eisessig/Wasser chromatographiert.

Ausbeute: 137 mg
FAB-MS 540 $(M^{+}H)^{+}$

Beispiel 2:

**(5-(3-Guanidinophenyl)-2,4-dioxoimidazolidin-3-yl)-acetyl-L-aspartyl-L-phenylglycin**

Diese Verbindung wurde analog der in Beispiel 1 beschriebenen Methode, ausgehend von (5-(3-Aminophenyl)-2,4-dioxo-imidazolidin-3-yl)-essigsäure, hergestellt.

FAB-MS 540 $(M^{+}H)^{+}$

Beispiel 3:

**(5-(4-Formamidinophenyl)-2,4-dioxo-imidazolidin-3-yl)-acetyl-L-aspartyl-L-phenylglycin**

Beispiel 4:

**(5-(4-Aminomethylphenyl)-2,4-dioxo-imidazolidin-3-yl)-acetyl-L-aspartyl-L-phenylglycin**

Beispiel 5:

**(5-(4-Formamidinophenyl)-4-oxo-2-thioxo-imidazolidin-3-yl)-acetyl-L-aspartyl-L-phenylglycin**

Beispiel 6:

**(5-(4-Formamidinophenyl)-4-oxo-2-thioxo-imidazolidin-3-yl)-acetyl-L-aspartyl-L-lysin**

Beispiel 7:

**(5-(4-Formamidinophenyl)-4-oxo-2-thioxo-imidazolidin-3-yl)-acetyl-L-aspartyl-L-valin**

Beispiel 8:

**(5-(4-Guanidinophenyl)-4-oxo-2-thioxo-imidazolidin-3-yl)-acetyl-L-aspartyl-L-phenylglycin**

Beispiel 9:

**(5-(4-Aminomethylphenyl)-2,4-dioxo-imidazolidin-3-yl)-acetyl-L-aspartyl-L-phenylalanin-(4-aminobutyl)-amid**

Beispiel 10:

**5-(4-Methoxycarbonyl-guanidinophenyl)-2,4-dioxoimidazolidin-3-yl-acetyl-L-aspartyl-(OMe)-L-phenylglycin-methylester**

Beispiel 11:

**(5-(R)-(4-Aminomethyl-phenyl)-2,4-dioxoimidazolidin-3-yl) -acetyl-L-aspartyl-L-phenyl glycin**

**a) 4-Benzyloxycarbonyl-aminomethyl-D-phenylglycin**

1 g 4-Aminomethyl-D-phenylglycin werden in 7 ml Wasser gelöst und mit 1.1 g $CuCO_3.Cu(OH)_2.0.5H_2O$ versetzt. Man kocht 45 min am Rückfluß, läßt abkühlen, stellt mit 2N Natronlauge alkalisch (über pH 9), gibt 0.13 ml Benzyloxycarbonylchlorid zu und läßt bei 0°C unter pH-Kontrolle 2N Natronlauge unter Rühren zutropfen. Der pH sollte nicht unter pH 9 sinken. Hat alles Benzyloxycarbonylchlorid abreagiert (keine pH-Änderung), wird abgesaugt.

Ausbeute: 1.74 g.

Der Niederschlag wird in ca. 40 ml 1N HCL bei ca. 55°C gelöst. Bei 50-60°C leitet man $H_2S$ ein bis sich die Lösung entfärbt hat. Man filtriert das CuS ab und neutralisiert die Lösung mit 10-proz. $NH_3$-Lösung. Hierbei fällt ein Niederschlag aus, der abgesaugt und nacheinander mit Wasser, Ethanol und Ether gewaschen wird.

Ausbeute: 300 mg.

**b) 4-Benzyloxycarbonyl-aminomethyl-D-phenylglycinmethylester-hydrochlorid**

300 mg 4-Benzyloxycarbonyl-aminomethyl-D-phenylglycin werden in 3 ml Methanol suspendiert und bei 0°C mit 97µl $SOCl_2$ versetzt. Man erhitzt 4 Stunden unter Rühren auf 40°C. Anschließend wird im Vakuum eingeengt und der Rückstand mit Ether verrieben.

Ausbeute: 292 mg.

**c) N-(1-(R)-(4-Benzyloxycarbonyl-aminomethyl-phenyl) -1-methoxycarbonylmethyl)-N'-ethoxycarbonylmethyl-harnstoff**

290 mg 4-Benzyloxycarbonyl-aminomethyl-D-phenylglycinmethylester-hydrochlorid werden in 1.5 ml Dimethylformamid gelöst. Dazu gibt man bei 0°C nacheinander 99 µl Isocyanatoessigsäureethylester und 122 µl Triethylamin. Mit wenig Triethylamin stellt man auf pH 8. Man läßt auf Raumtemperatur kommen und destilliert am nächsten Tag das Dimethylformamid im Vakuum ab. Der Rückstand wird zwischen Essigester und Wasser verteilt, die Essigesterphase abgetrennt und mit $KHSO_4/K_2SO_4$-Puffer, gesättigter $NaHCO_3$-Lösung und Wasser extrahiert, über $Na_2SO_4$ getrocknet und eingeengt.

Ausbeute: 310 mg.

**d) (5-(R)-(4-Aminomethyl-phenyl)2,4-dioxoimdazolidin-3-yl)-essigsäurehydrochlorid**

280 mg N-(1-(R)-(4-Benzyloxycarbonyl-aminomethylphenyl)-1-methoxycarbonylmethyl, N'ethoxycarbonylmethyl-harnstoff werden in 4 ml 6N HCl 45 min am Rückfluß gekocht und anschließend eingeengt und über KOH getrocknet.

Ausbeute: 180 mg.

**e) (5-(R)-(4-tert.-Butyloxycarbony)-aminomethyl-phenyl) -2,4-dioxoimidazolidin-3-yl)-essigsäure**

Eine Lösung von 180 mg (0.6 mmol) (5-(R)-(4-Aminomethyl-phenyl)-2,4-dioxoimidazolidin-3-yl)-essigsäure-hydrochlorid in einer Mischung aus 2 ml Dioxan und 1 ml Wasser wird bei 0°C mit ca. 1 ml 1N NaOH auf pH 8-9 gestellt. Dazu gibt man 142 mg Di-tert-butyldicarbonat und läßt 3 Stunden bei Raumtemperatur rühren. Danach wird der Ansatz einrotiert und der Rückstand zwischen Essigester und Wasser, das mit $KHSO_4$ auf pH 2 angesäuert wurde, verteilt. Die Essigesterphase wird nun zweimal mit gesättigtor $NaHCO_3$-Lösung ausgeschüttelt. Die vereinigten $NaHCO_3$-Lösungen werden mit $KHSO_4$ auf pH 2 angesäuert und dreimal mit Essigester extrahiert. Die vereinigten Essigesterphasen werden mit Wasser gewaschen, über $Na_2SO_4$ getrocknet und eingeengt.

Ausbeute: 180 mg.

**f) (5-(R)-(4-tert.-Butyloxycarbonyl-aminomethyl-phenyl)-2,4-dioxoimidazolidin-3-yl)-acetyl-L-aspartyl-L-phenylglycin-di-tert.-butylester**

Zu einer Suspension von 160 mg (0.44 mmol)(5-(R)-(4-tert. -Butyloxycarbonylaminomethyl-phenyl)-2,4-dioxoimidazolidin-3-yl)-essigsäure, 183 mg H-Asp(OtBu)-Phg-OtBu-hydrochlorid und 60 mg HOBt in 10 ml Dimethylformamid gibt man bei 0°C 55.5 µl N-Ethylmorpholin und 97 mg Dicyclohexylcarbodiimid. Man rührt 1 Stunde bei 0°C und 3 Stunden bei Raumtemperatur. Danach wird der Niederschlag abgesaugt und das Filtrat eingeengt. Der Rückstand wird zwischen Essigester und Wasser verteilt, die Essigesterphase abgetrennt und mit $KHSO_4/K_2SO_4$-Puffer, gesättigter $NaHCO_3$-Lösung und Wasser extrahiert, über $Na_2SO_4$ getrocknet und eingeengt.

Ausbeute: 340 mg.

**g) (5-(R)-(4-Aminomethyl-phenyl)-2,4-dioxoimidazolidin-3-yl)-acetyl-L-aspartyl-L-phenylglycin**

310 mg (5-(R)-(4-tert.-Butyloxycarbonyl-aminomethyl-phenyl)-2,4-dioxoimidazolidin-3-yl)-acetyl-L-aspartyl-L-phenylglycin-di-tert.-butylester werden in 3.1 ml 90%iger wässriger Trifluoressigsäure gelöst. Man läßt 45 min bei Raumtemperatur stehen, engt ein und verteilt den Rückstand zwischen Wasser und Ether. Die wässrige Phase wird gefriergetrocknet.

Ausbeute: 200 mg.

Zur Reinigung wird die Substanz an Sephadex LH20 in Wasser chromatographiert.

Ausbeute: 180 mg.
FAB-MS: 512.2 $(M+H)^+$

Beispiel 12:

**5-(4-Guanidinophenyl)-2,4-dioxo-imidazolidin-3-yl)-acetyl -D-aspartyl-L-phenylglycin**

FAB-MS: 540.2 $(M+H)^+$

Beispiel 13:

**5-(4-Benzyloxycarbonylguanidino-phenyl)-2,4-dioxo-imidazolidin-3-yl)-acetyl-L-aspartyl-L-phenylglycin-diisopropylester**

Zu einer Suspension von 1.2 g (2.8 mmol) 5-(4-Benzyloxycarbonylguanidino-phenyl)-2,4-dioxo-imidazolidin-3-yl)-essigsäure und 380 mg HOBt in 180 ml Dimethylformamid gibt man bei 0°C 620 mg Dicyclohexylcarbodiimid und rührt 2 Stunden bei 0°C. Dann werden 1.28 g (3.3 mmol) H-Asp-Phg-diisopropylester-hydrochlorid und 640 mg N-Ethylmorpholin zugegeben. Man rührt 1 Stunde bei 0°C und 3 Stunden bei Raumtemperatur. Danach wird der Niederschlag abgesaugt und das Filtrat eingeengt. Der Rückstand wird zwischen Essigester und Wasser verteilt, die Essigesterphase abgetrennt und mit $KHSO_4/K_2SO_4$-Puffer, gesättigter $NaHCO_3$-Lösung und Wasser extrahiert, über $Na_2SO_4$ getrocknet und eingeengt.

Ausbeute: 2.1 g.
Schmelzpunkt: ≈ 110°C
FAB-MS: 758.3 $(M+H)^+$

Beispiel 14

**5-(4-Guanidinophenyl)-2,4-dioxo-imidazolidin-3-yl)-acetyl -L-aspartyl-L-phenylglycin-diisopropylester-hydrochlorid**

1.0 g (1.32 mmol) 5-(4-Benzyloxycarbonylguanidino-phenyl)-2,4-dioxo-imidazolidin-3-yl)-acetyl-L-aspartyl-L-phenylglycin-diisopropylester werden in 200 ml Methanol gelöst und in Gegenwart von 0.1 g 10% Pd/C bei Raumtemperatur hydriert. Der pH-Wert wird dabei durch Zutropfen von methanolischer Salzsäure auf pH 4 gehalten.

Nach Ende der Hydrierung wird vom Katalysator abfiltriert und eingeengt.

Ausbeute: 850 mg
FAB-MS: 624.2 $(M+H)^+$

Analog dieser Beispiele können die folgenden Verbindungen hergestellt werden:

Beispiel 15:

**5-(4-Guanidinophenyl)-2,4-dioxo-imidazolidin-3-yl)-acetyl -L-aspartyl-L-phenylglycin-diethylester-hydrochlorid**

Schmelzpunkt: ≈ 150°C
FAB-MS: 596.3 $(M+H)^+$

Beispiel 16:

**5-(4-Di-(methoxycarbonyl)guanidino-phenyl)-2,4-dioxo-imidazolidin-3-yl)-acetyl-L-aspartyl-L-phenylglycin-diethylester**

Schmelzpunkt: ≈ 100°C
FAB-MS: 712.3 $(M+H)^+$

Beispiel 17:

**5-(4-Benzyloxycarbonylguanidino-phenyl)-2,4-dioxo-imidazolidin-3-yl)-acetyl-L-aspartyl-L-phenylglycin-diisobutylester**

Schmelzpunkt: ≈ 110°C
FAB-MS: 786.7 $(M+H)^+$

Beispiel 18:

**5-(4-Guanidinophenyl)-2,4-dioxo-imidazolidin-3-yl)-acetyl -L-aspartyl-L-phenylglycin-diisobutylester-acetat**

Schmelzpunkt: >200°C (Zers.)
FAB-MS: 652.3 $(M+H)^+$

Beispiel 19:

**5-(4-Benzyloxycarbonylguanidino-phenyl)-2,4-dioxo-imidazolidin-3-yl)-acetyl-L-aspartyl-L-phenylglycin-dimethylester**

FAB-MS: 702.3 $(M+H)^+$

Beispiel 20:

**5-(4-Guanidinophenyl)-2,4-dioxo-imidazolidin-3-yl)-acetyl -L-aspartyl-L-phenylglycin-dimethylester-hydrochlorid**

FAB-MS: 568.2 $(M+H)^+$

Beispiel 21:

**5-(4-Benzyloxycarbonylguanidino-phenyl)-2,4-dioxo-imidazolidin-3-yl)-acetyl-L-aspartyl-L-phenylglycin**

FAB-MS: 674.3 $(M+H)^+$

Beispiel 22:

**5-(4-Benzyloxycarbonylguanidino-phenyl)-2,4-dioxo-imidazolidin-3-yl)-acetyl-L-aspartyl-L-phenylglycin-methylester**

Schmelzpunkt: ≈165°C
FAB-MS: 688.5 $(M+H)^+$

Beispiel 23:

**5-(4-Methoxycarbonylguanidino-phenyl)-2,4-dioxo-imidazolidin-3-yl)-acetyl-L-aspartyl-L-phenylglycin**

FAB-MS: 598.3 $(M+H)^+$

Beispiel 24:

**5-(4-Methoxycarbonylguanidino-phenyl)-2,4-dioxo-imidazolidin-3-yl)-acetyl-L-aspartyl-L-phenylglycin-dimethylester**

FAB-MS:626.2 $(M+H)^+$

Beispiel 25:

**5-(4-Methoxycarbonylguanidino-phenyl)-2,4,-dioxo-imidazolidin-3-yl)-acetyl-L-aspartyl(O-isopropyl)-L-phenylglycin-(1-hexadecylester)**

FAB-MS: 865.3 $(M+H)^+$

Beispiel 26:

**5-(4-Acetylguanidino-phenyl)-2,4-dioxo-imidazolidin-3-yl) -acetyl-L-aspartyl-L-phenylglycin-diisopropylester**

FAB-MS: 666.3 $(M+H)^+$

Beispiel 27:

**(5-(4-Aminomethyl-phenyl)-2,4-dioxoimidazolidin-3-yl)-acetyl-L-aspartyl-L-phenylglycin-diisopropylester-hydrochlorid**

FAB-MS: 596.2 $(M+H)^+$

Beispiel 28.

**(5-(4-Acetylaminomethyl-phenyl)-2,4-dioxoimidazolidin-3-yl)-acetyl-L-aspartyl-L-phenylglycin-diisopropylester**

FAB-MS: 638.2 $(M+H)^+$

Beispiel 29:

**(5-(4-Methoxycarbonylaminomethyl-phenyl)-2,4-dioxoimidazolidin-3-yl)-acetyl-L-aspartyl-L-phenylglycin-diisopropylester**

FAB-MS: 654.2 $(M+H)^+$

Beispiel 30:

**(5-(4-Aminomethyl-phenyl)-2,4-dioxoimidazolidin-3-yl) -acetyl-L-aspartyl(O-isopropyl)-L-phenylglycin-(2-(2-(2-hydroxyethoxy)-ethoxy)-ethoxy)-ethylesterhydrochlorid**

FAB-MS: 730.3 $(M+H)^+$

Die nachfolgenden Beispiele A - H betreffen pharmazeutische Zubereitungen

Beispiel A

Emulsionen mit 3 mg Wirkstoff per 5 ml können nach folgender Rezeptur hergestellt werden:

| | |
|---|---|
| Wirkstoff | 0,06 g |
| Neutralöl | q. s. |
| Natriumcarboxymethylzellulose | 0,6 g |
| Polyoxyethylenstearat | q. s. |
| Reinglycerin | 0,6 bis 2 g |
| Aromastoffe | q. s. |

(fortgesetzt)

| | |
|---|---|
| Wasser (entmineralisiert oder destilliert) | ad 100 ml |

Beispiel B

Tabletten können nach folgender Formulierung hergestellt werden:

| | |
|---|---|
| Wirkstoff | 2 mg |
| Lactose | 60 mg |
| Maisstärke | 30 mg |
| lösliche Stärke | 4 mg |
| Magnesiumstearat | 4 mg |
| | 100 mg |

Beispiel C

Für die Herstellung von Weichgelatinekapseln mit 5 mg Wirkstoff pro Kapsel eignet sich die folgende Zusammensetzung:

| | |
|---|---|
| Wirkstoff | 5 mg |
| Mischung von Triglyceriden aus Kokosöl | 150 mg |
| Kapselinhalt | 155 mg |

Beispiel D

Für die Herstellung von Dragees eignet sich folgende Formulierung:

| | |
|---|---|
| Wirkstoff | 3 mg |
| Maisstärke | 100 mg |
| Lactose | 55 mg |
| sec. Calciumphosphat | 30 mg |
| lösliche Stärke | 3 mg |
| Magnesiumstearat | 5 mg |
| kolloidale Kieselsäure | 4 mg |
| | 200 mg |

Beispiel E

Dragees, enthaltend einen erfindungsgemäßen Wirkstoff und einen anderen therapeutisch wirksamen Stoff:

| | |
|---|---|
| Wirkstoff | 6 mg |
| Propanolol | 40 mg |
| Milchzucker | 90 mg |
| Maisstärke | 90 mg |
| sec. Calciumphosphat | 34 mg |
| lösliche Stärke | 3 mg |
| Magnesiumstearat | 3 mg |
| kolloidale Kieselsäure | 4 mg |
| | 270 mg |

Beispiel F

Dragees, enthaltend einen erfindungsgemäßen Wirkstoff und einen anderen therapeutisch wirksamen Stoff:

| | |
|---|---|
| Wirkstoff | 5 mg |
| Pirlindol | 5 mg |
| Milchzucker | 60 mg |
| Maisstärke | 90 mg |
| sec. Calciumphosphat | 30 mg |
| lösliche Stärke | 3 mg |
| Magnesiumstearat | 3 mg |
| kolloidale Kieselsäure | 4 mg |
| | 200 mg |

Beispiel G

Kapseln, enthaltend einen erfindungsgemäßen Wirkstoff und einen anderen therapeutisch wirksamen Stoff:

| | |
|---|---|
| Wirkstoff | 5 mg |
| Nicergolin | 5 mg |
| Mais- stärke | 185 mg |
| | 195 mg |

Beispiel H

Injektionslösungen mit 1 mg Wirkstoff pro ml können nach folgender Rezeptur hergestellt werden:

| | |
|---|---|
| Wirkstoff | 1,0 mg |
| Polyethylenglykol 400 | 0,3 mg |
| Natriumchlorid | 2,7 mg |
| Wasser zu Injektionszwecken auf | 1 ml |

**Patentansprüche**

**1.** Phenylimidazolidin-Derivate der allgemeinen Formel I,

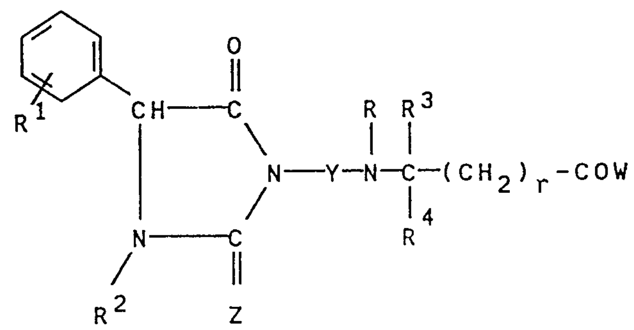

(I)

worin

Y $-(CH_2)_m-CO-$, wobei m für eine Zahl von 1 bis 4 steht, oder

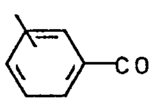

bedeutet;

r eine Zahl von 0 bis 3 bedeutet;

Z Sauerstoff oder Schwefel bedeutet;

W Hydroxy, $(C_1-C_{28})$-Alkoxy, $(C_6-C_{14})$-Aryl-$(C_1-C_8)$-alkoxy, das im Arylrest auch substituiert sein kann, gegebenenfalls substituietes $(C_6-C_{14})$-Aryloxy, Amino oder Mono-oder Di-$(C_1-C_{18})$-Alkyl-amino bedeutet;

$R^1$ für $-(CH_2)_n-NH-X$ oder $-(CH_2)_p-C(=NH)-NH-X^1$, wobei n und p für eine Zahl 0 bis 3 stehen, bedeutet;

$X,X^1$ Wasserstoff, $(C_1-C_6)$-Alkyl, $(C_1-C_6)$-Alkoxycarbonyl, $(C_1-C_6)$-Alkylcarbonyl, $(C_1-C_{18})$-Alkylcarbonyl-oxy-$(C_1-C_6)$-alkoxycarbonyl, $(C_6-C_{14})$-Aryloxycarbonyl, das im Arylrest auch substituiert sein kann, $(C_6-C_{14})$-Aryl$(C_1-C_6)$-alkoxycarbonyl, das im Arylrest auch substituiert sein kann, Cyano, Hydroxy, $(C_1-C_6)$-Alkoxy oder Amino steht, und

X zusätzlich einen Rest der Formel II

$$R'-NH-C(=N-R'')- \quad (II)$$

wobei

R' und R'' unabhängig voneinander für Wasserstoff, $(C_1-C_6)$-Alkyl, $(C_1-C_6)$-Alkoxycarbonyl, $(C_1-C_6)$-Alkylcarbonyl, $(C_1-C_{18})$-Alkylcarbonyl-oxy-$(C_1-C_6)$-alkoxycarbonyl, $(C_6-C_{14})$-Aryloxycarbonyl, das im Arylrest auch substituiert sein kann, $(C_6-C_{14})$-Aryl$(C_1-C_6)$-alkoxycarbonyl, das im Arylrest auch substituiert sein kann, Cyano, Hydroxy, $(C_1-C_6)$-Alkoxy oder Amino stehen, bedeutet;

R, $R^2$ Wasserstoff oder $(C_1-C_6)$-Alkyl bedeutet;

$R^3$ Wasserstoff, Phenyl oder substituiertes Phenyl bedeutet;

$R^4$ Wasserstoff, $-COOR^5$, $-CO-N(CH_3)-R^5$ oder $-CO-NH-R^5$ bedeutet;

$R^5$ Wasserstoff oder $(C_1-C_{28})$-Alkyl bedeutet, das gegebenenfalls ein- oder mehrfach durch gleiche oder verschiedene Reste aus der Reihe Hydroxy, Hydroxycarbonyl, Aminocarbonyl, Mono- oder Di-$(C_1-C_{18})$-alkylaminocarbonyl, Amino-$(C_2-C_{14})$-alkylaminocarbonyl, Amino$(C_1-C_3)$-alkylphenyl-$(C_1-C_3)$-alkylaminocarbonyl, $(C_1-C_{18})$ -Alkylcarbonylamino-$(C_1-C_3)$-alkylphenyl-$(C_1-C_3)$-alkylaminocarbonyl, $(C_1-C_{18})$-Alkylcarbonylamino-$(C_2-C_{14})$-alkylaminocarbonyl, Phenyl-$(C_1-C_8)$-alkoxycarbonyl, das im Arylrest auch substituiert sein kann, Amino, Mercapto, $(C_1-C_{18})$-Alkoxy, $(C_1-C_{18})$-Alkoxycarbonyl, gegebenenfalls substituiertes $(C_3-C_8)$-Cycloalkyl, Halogen, Nitro, Trifluormethyl oder einen Rest $R^6$ substituiert ist, wobei

$R^6$ für gegebenenfalls substituiertes $(C_6-C_{14})$-Aryl, gegebenenfalls substituiertes $(C_6-C_{14})$-Aryl-$(C_1-C_8)$-alkyl oder einen mono- oder bi zyklischen 5- bis 12-gliedrigen heterozyklischen Ring, der aromatisch, teilhydriert oder vollständig hydriert sein kann und der als Heteroelement ein, zwei oder drei gleiche oder verschiedene Stickstoff-, Sauerstoff- oder Schwefel-Atome enthalten kann, oder einen Rest $R^7$ bedeutet wobei der Aryl- und unabhängig voneinander der Heterozyklus-Rest gegebenfalls ein- oder mehrfach durch gleiche oder verschiedene Reste aus der Reihe $(C_1-C_{18})$-Alkyl, $(C_1-C_{18})$-Alkoxy, Halogen, Nitro, Amino oder Trifluormethyl substituiert sein können;

$R^7$ $-NR^8R^9$, $-OR^8$, $-SR^8$, eine Aminosäureseitenkette, einen natürlichen oder unnatürlichen Aminosäure-, Iminosäure-, gegebenenfalls N-$(C_1-C_8)$-alkylierten oder $(C_6-C_{14})$-Aryl-$(C_1-C_8)$-alkylierten Azaaminosäure- oder einen Dipeptid-Rest, der im Arylrest auch substituiert und/oder bei dem die Peptidbindung zu $-NH-CH_2-$ reduziert sein kann, sowie deren Ester und Amide, wobei freie funktionelle Gruppen gegebenfalls durch Wasserstoff oder Hydroxymethyl substituiert oder durch in der Peptidchemie übliche Schutzgruppen geschützt sein können, oder einen Rest $-COR^{7'}$ bedeuten, worin $R^{7'}$ wie $R^7$ definiert ist, bedeutet;

$R^8$ Wasserstoff, $(C_2-C_{18})$-Alkyl, gegebenenfalls substituiertes $(C_6-C_{14})$-Aryl-$(C_1-C_8)$alkyl, $(C_1-C_{18})$-Alkylcarbonyl, $(C_1-C_{18})$-Alkoxycarbonyl, $(C_6-C_{14})$-Arylcarbonyl, $(C_6-C_{14})$-Aryl-$(C_1-C_8)$-alkylcarbonyl oder $(C_6-C_{14})$-Aryl-$(C_1-C_{18})$-alkyloxycarbonyl, wobei die Alkylgruppen gegebenenfalls durch eine Aminogruppe und/oder wobei die Arylreste ein- oder mehrfach vorzugsweise einfach durch gleiche oder verschiedene Reste aus der Reihe $(C_1-C_8)$-Alkyl, $(C_1-C_8)$-Alkoxy, Halogen, Nitro, Amino oder Trifluormethyl substituiert sein können, einen natürlichen oder unnatürlichen Aminosäure-, Iminosäure-, gegebenenfalls N-$(C_1-C_8)$-alkylierten oder $(C_6-C_{14})$-Aryl-$(C_1-C_8)$-alkylierten Azaaminosäure- oder einen Dipeptid-Rest, der im Arylrest auch substituiert und/oder bei dem die Peptidbindung zu $-NH-CH_2-$ reduziert sein kann, bedeutet; und

R9 Wasserstoff, $(C_1-C_{18})$-Alkyl, gegebenenfalls substituiertes $(C_6-C_{14})$-Aryl oder $(C_6-C_{14})$-Aryl-$(C_1-C_8)$-alkyl, das im Arylrest auch substituiert sein kann, bedeutet;

sowie deren physiologisch verträgliche Salze.

2. Phenylimidazolidin-Derivate nach Anspruch 1, dadurch gekennzeichnet, daß Y $-(CH_2)_m-CO-$, wobei m für 1 oder 2 steht, oder

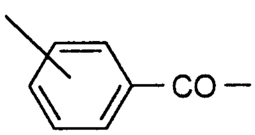

bedeutet;

r 1 bedeutet;

Z Sauerstoff oder Schwefel bedeutet;

W Hydroxy oder $(C_1-C_6)$-Alkoxy, besonders Methoxy, Ethoxy oder 2-Propyloxy, bedeutet;

R Wasserstoff bedeutet;

$R^1$ $-NH-C(=NH)-NH_2$, $-C(=NH)-NH_2$ oder $-CH_2-NH_2$ oder Methoxycarbonylderivate davon bedeutet;

$R^2$ Wasserstoff oder Methyl bedeutet;

$R^3$ Wasserstoff bedeutet; und

$R^4$ $-CO-NH-R^5$ bedeutet, wobei $-NH-R^5$ für einen $\alpha$-Aminosäurerest oder dessen x-Amino-$(C_2-C_8)$-alkylamid steht.

3. Phenylimidazolidin-Derivate nach Anspruch 1 und/oder 2, dadurch gekennzeichnet, daß

   $R^4$ -CO-NH-$R^5$ bedeutet, wobei -NH-$R^5$ für den Valin-, Lysin-, Phenylalanin- oder Phenylglycin-Rest steht.

4. Phenylimidazolidin-Derivate nach einem oder mehreren der Ansprüche 1 bis 3, dadurch gekennzeichnet, daß $R^4$ -CO-NH-$R^5$ bedeutet, wobei $R^5$ für das 4-Aminobutylamid einer $\alpha$-Aminosäure steht.

5. Verfahren zur Herstellung der Phenylimidazolidin-Derivate der Formel I eines oder mehreren der Ansprüche 1 bis 4, dadurch gekennzeichnet, daß eine Fragmentkondensation einer Verbindung der allgemeinen Formel III

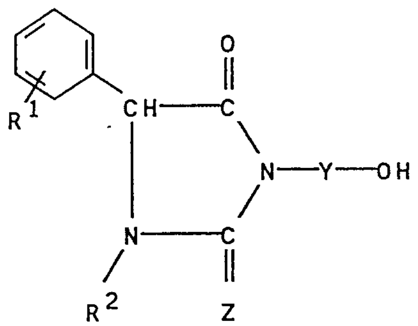

(III)

mit einer Verbindung der allgemeinen Formel IV

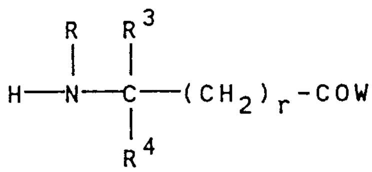

(IV)

wobei r und die Reste R, $R^1$ bis $R^4$, Z, Y und W die in einem oder mehreren der Ansprüche 1 bis 4 gegebenen Definitionen besitzen, durchgeführt wird.

6. Verbindung der allgemeinen Formel I gemäß einem oder mehreren der Ansprüche 1 bis 4 sowie deren physiologisch verträgliche Salze zur Anwendung als Hemmstoff der Thrombozytenaggregation, von Thrombosen, der Metastasierung von Karzinomzellen oder der Osteoclastenbindung an die Knochenoberflächen.

7. Pharmazeutisches Präparat, dadurch gekennzeichnet, daß es eine oder mehrere Verbindungen der allgemeinen Formel I gemäß einem oder mehreren der Ansprüche 1 bis 4 oder ein physiologisch verträgliches Salz davon als Wirkstoff zusammen mit pharmazeutisch annehmbaren Träger- und Zusatzstoffen und gegebenenfalls noch ein oder mehrere andere pharmakologische Wirkstoffe enthält.

8. Verfahren zur Herstellung eines pharmazeutischen Präparates, enthaltend eine oder mehrere Verbindungen der allgemeinen Formel I gemäß einem oder mehreren der Ansprüche 1 bis 4 oder ein physiologisch verträgliches Salz davon, dadurch gekennzeichnet, daß man diese zusammen mit pharmazeutisch annehmbaren Träger- und Zusatzstoffen und gegebenenfalls noch einem oder mehreren anderen pharmakologischen Wirkstoffen in eine geeignete Darreichungsform bringt.

## Claims

1. A phenylimidazolidine derivative of the formula I

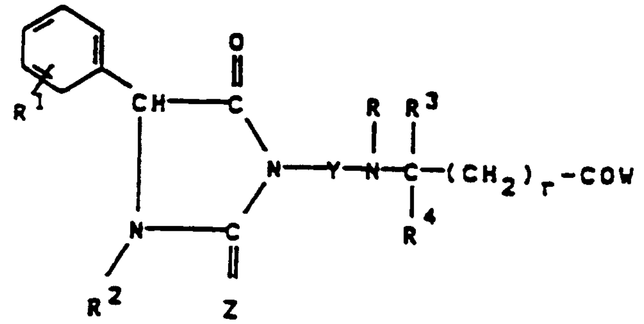

(I)

in which

Y is $-(CH_2)_m-CO-$, where m is a number from 1 to 4, or

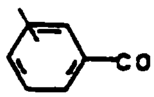

r is a number from 0 to 3;

Z is oxygen or sulfur;

W is hydroxyl, $(C_1-C_{28})$-alkoxy, $(C_6-C_{14})$-aryl-$(C_1-C_8)$-alkoxy which can also be substituted in the aryl radical, optionally substituted $(C_6-C_{14})$-aryloxy, amino or mono- or di-$(C_1-C_{18})$-alkyl-amino;

$R^1$ is $-(CH_2)_n-NH-X$ or $-(CH_2)_p-C(=NH)-NH-X^1$, where n and p are a number 0 to 3;

X,$X^1$ are hydrogen, $(C_1-C_6)$-alkyl, $(C_1-C_6)$-alkoxycarbonyl, $(C_1-C_6)$-alkylcarbonyl, $(C_1-C_{18})$-alkylcarbonyloxy-$(C_1-C_6)$-alkoxycarbonyl, $(C_6-C_{14})$-aryloxycarbonyl which can also be substituted in the aryl radical, $(C_6-C_{14})$-aryl-$(C_1-C_6)$-alkoxycarbonyl which can also be substituted in the aryl radical, cyano, hydroxyl, $C_1-C_6$-alkoxy or amino, and

X is additionally a radical of the formula II

$$R'-NH-C(=N-R'')- \qquad (II)$$

where

R' and R" independently of one another are hydrogen, $(C_1-C_6)$-alkyl, $(C_1-C_6)$-alkoxycarbonyl, $(C_1-C_6)$-alkylcarbonyl, $(C_1-C_{18})$-alkylcarbonyloxy-$(C_1-C_6)$-alkoxycarbonyl, $(C_6-C_{14})$-aryloxycarbonyl which can also be substituted in the aryl radical, $(C_6-C_{14})$-aryl-$(C_1-C_6)$-alkoxycarbonyl which can also be substituted in the aryl radical, cyano, hydroxyl, $(C_1-C_6)$-alkoxy or amino;

R, $R^2$ are hydrogen or $(C_1-C_6)$-alkyl;

$R^3$ is hydrogen, phenyl or substituted phenyl;

$R^4$ is hydrogen, $-COOR^5$, $-CO-N(CH_3)-R^5$ or $-CO-NH-R^5$;

$R^5$ is hydrogen or $(C_1-C_{28})$-alkyl which is optionally mono- or polysubstituted by identical or different radicals from the group consisting of hydroxyl, hydroxycarbonyl, aminocarbonyl, mono- or di-$(C_1-C_{18})$-alkylaminocarbonyl, amino-$(C_2-C_{14})$-alkylaminocarbonyl, amino$(C_1-C_3)$-alkylphenyl-$(C_1-C_3)$-alkylaminocarbonyl, $(C_1-C_{18})$-alkylcarbonylamino-$(C_1-C_3)$-alkylphenyl-$(C_1-C_3)$-alkylaminocarbonyl, $(C_1-C_{18})$-alkylcarbonylamino-$(C_2-C_{14})$-alkylaminocarbonyl, phenyl-$(C_1-C_8)$-alkoxycarbonyl which can also be substituted in the aryl radical, amino, mercapto, $(C_1-C_{18})$-alkoxy, $(C_1-C_{18})$-alkoxycarbonyl, optionally substituted $(C_3-C_8)$-cycloalkyl, halogen, nitro, trifluoromethyl or a radical $R^6$ where

$R^6$ is optionally substituted $(C_6-C_{14})$-aryl, optionally substituted $(C_6-C_{14})$-aryl-$(C_1-C_8)$-alkyl or a mono- or bicyclic 5- to 12-membered heterocyclic ring which can be aromatic, partially hydrogenated or completely hydrogenated and which as a hetero element can contain one, two or three identical or different nitrogen, oxygen or sulfur atoms, or is a radical $R^7$ where the aryl radical and, independently of one another, the heterocyclic radical can optionally be mono- or polysubstituted by identical or different radicals from the group consisting of $(C_1-C_{18})$-alkyl, $(C_1-C_{18})$-alkoxy, halogen, nitro, amino or

trifluoromethyl;

$R^7$ is $-NR^8R^9$, $-OR^8$, $-SR^8$, an amino acid side chain, a natural or unnatural amino acid radical, imino acid radical, optionally N-($C_1$-$C_8$)-alkylated or ($C_6$-$C_{14}$)-aryl-($C_1$-$C_8$)-alkylated azaamino acid radical or a dipeptide radical which can also be substituted in the aryl radical and/or in which the peptide bonds can be reduced to $-NH-CH_2-$, and their esters and amides, where free functional groups can optionally be substituted by hydrogen or hydroxymethyl or protected by protective groups customary in peptide chemistry, or is a radical $-COR^{7'}$ in which $R^{7'}$ is defined as $R^7$;

$R^8$ is hydrogen, ($C_2$-$C_{18}$)-alkyl, optionally substituted ($C_6$-$C_{14}$)-aryl-($C_1$-$C_8$)-alkyl, ($C_1$-$C_{18}$)-alkylcarbonyl, ($C_1$-$C_{18}$)-alkoxycarbonyl, ($C_6$-$C_{14}$)-arylcarbonyl, ($C_6$-$C_{14}$)aryl-($C_1$-$C_8$)-alkylcarbonyl or ($C_6$-$C_{14}$)aryl-($C_1$-$C_{18}$)-alkoxycarbonyl, where the alkyl groups can optionally be substituted by an amino group and/or where the aryl radicals can be mono- or polysubstituted, preferably mono-substituted by identical or different radicals from the group consisting of ($C_1$-$C_8$)-alkyl, ($C_1$-$C_8$)-alkoxy, halogen, nitro, amino or trifluoromethyl, a natural or unnatural amino acid radical, imino acid radical, optionally N-($C_1$-$C_8$)-alkylated or ($C_6$-$C_{14}$)-aryl-($C_1$-$C_8$)-alkylated azaamino acid radical or a dipeptide radical which can also be substituted in the aryl radical and/or in which the peptide bond can be reduced to $-NH-CH_2-$; and

$R^9$ is hydrogen, ($C_1$-$C_{18}$)-alkyl, optionally substituted ($C_6$-$C_{14}$)-aryl or ($C_6$-$C_{14}$)-aryl-($C_1$-$C_8$)-alkyl which can also be substituted in the aryl radical;

or its physiologically tolerated salts.

**2.** A phenylimidazolidine derivative as claimed in claim 1, wherein

Y is $-(CH_2)_m$-CO-, where m is 1 or 2, or

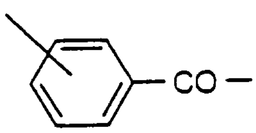

r is 1;
Z is oxygen or sulfur;
W is hydroxyl or ($C_1$-$C_6$)-alkoxy, particularly methoxy, ethoxy or 2-propyloxy;
R is hydrogen;
$R^1$ is $-NH-C(=NH)-NH_2$, $-C(=NH)-NH_2$ or $-CH_2-NH_2$ or methoxycarbonyl derivatives thereof;
$R^2$ is hydrogen or methyl;
$R^3$ is hydrogen; and
$R^4$ is $-CO-NH-R^5$, where $-NH-R^5$ is an $\alpha$-amino acid radical or its x-amino-($C_2$-$C_8$)-alkylamide.

**3.** A phenylimidazolidine derivative as claimed in claim 1 and/or 2, wherein

$R^4$ is $-CO-NH-R^5$, where $-NH-R^5$ is the valine, lysine, phenylalanine or phenylglycine radical.

**4.** A phenylimidazolidine derivative as claimed in one or more of claims 1 to 3, wherein $R^4$ is $-CO-NH-R^5$, where $R^5$ is the 4-aminobutylamide of an $\alpha$-amino acid.

**5.** A process for the preparation of the phenylimidazolidine derivatives of the formula I of one or more of claims 1 to 4, which comprises carrying out a fragment condensation of a compound of the formula III

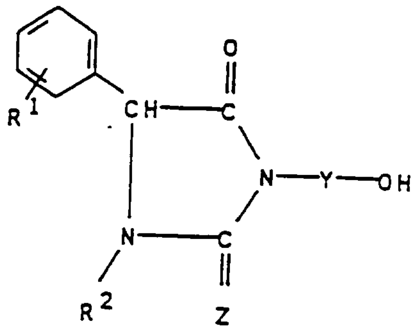

(III)

with a compound of the formula IV

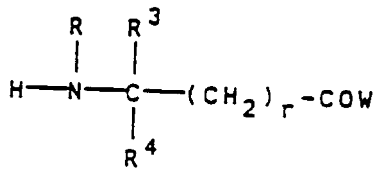

(IV)

where r and the radicals, R, $R^1$ to $R^4$, Z, Y and W have the definitions given in one or more of claims 1 to 4.

6. A compound of the formula I as claimed in one or more of claims 1 to 4, or its physiologically tolerable salts for use as an inhibitor of platelet aggregation, of thromboses, of the metastasis of carcinoma cells or of osteoclast binding to the bone surfaces.

7. A pharmaceutical preparation, which comprises one or more compounds of the formula I as claimed in one or more of claims 1 to 4 or a physiologically tolerable salt thereof as an active compound together with pharmaceutically acceptable excipients and additives and, if appropriate, additionally one or more other pharmacological active substances.

8. A process for the production of a pharmaceutical preparation, comprising one or more compounds of the formula I as claimed in one or more of claims 1 to 4 or a physiologically tolerable salt thereof, which comprises bringing these into a suitable administration form together with pharmaceutically acceptable excipients and additives and, if appropriate, additionally one or more other pharmacological active compounds.

**Revendications**

1. Dérivés de phénylimidazolidine de formule générale I,

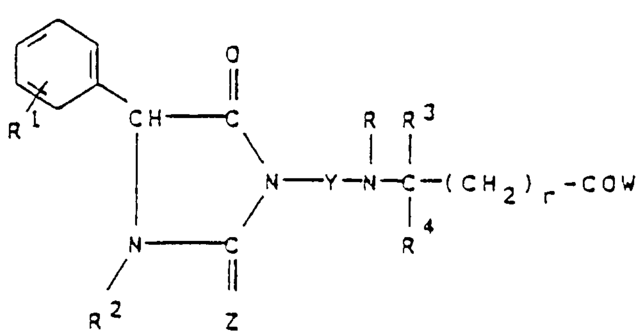

(I)

dans laquelle

Y représente $-(CH_2)_m-CO-$, m représentant un nombre entier allant de 1 à 4, ou

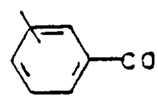

r représente un nombre allant de 0 à 3;

Z représente un atome d'oxygène ou de soufre;

W représente un groupe hydroxyle, alcoxy en $C_1-C_{28}$, aryl($C_6-C_{14}$)-alcoxy($C_1-C_8$) qui peut également être substitué sur le fragment aryle, aryloxy en $C_6-C_{14}$ éventuellement substitué, amino ou mono- ou dialkyl($C_1-C_{18}$)-amino;

$R^1$ représente un groupe $-(CH_2)_n-NH-X$ ou $-(CH_2)_p-C(=NH)-NH-X^1$, n et p étant chacun un nombre entier allant de 0 à 3;

X, $X^1$ représentent un atome d'hydrogène ou un groupe alkyle en $C_1-C_6$, alcoxy($C_1-C_6$)-carbonyle, alkyl($C_1-C_6$)-carbonyle, alkyl($C_1-C_{18}$)-carbonyloxy-alcoxy($C_1-C_6$)-carbonyle, aryloxy($C_6-C_{14}$)-carbonyle qui peut également être substitué sur le fragment aryle, aryl($C_6-C_{14}$)-alcoxy($C_1-C_6$)-carbonyle qui peut également être substitué sur le fragment aryle, cyano, hydroxyle, alcoxy en $C_1-C_6$ ou amino, et

X représente en outre un radical de formule II

$$R'-NH-C(=N-R'')- \qquad (II),$$

dans laquelle

R', R'' représentent, indépendamment l'un de l'autre, un atome d'hydrogène ou un groupe alkyle en $C_1-C_6$, alcoxy($C_1-C_6$)-carbonyle, alkyl($C_1-C_6$)-carbonyle, alkyl ($C_1-C_{18}$)-carbonyloxy-alcoxy($C_1-C_6$)-carbonyle, aryloxy($C_6-C_{14}$)-carbonyle qui peut également être substitué sur le fragment aryle, un groupe aryl($C_6-C_{14}$)-alcoxy($C_1-C_6$)-carbonyle qui peut également être substitué sur le fragment aryle, ou un groupe cyano, hydroxyle, alcoxy en $C_1-C_6$ ou amino;

R, $R^2$ représentent un atome d'hydrogène ou un groupe alkyle en $C_1-C_6$;

$R^3$ représente un atome d'hydrogène ou un groupe phényle ou phényle substitué;

$R^4$ représente un atome d'hydrogène ou un groupe $-COOR^5$, $-CO-N(CH_3)-R^5$ ou $-CO-NH-R^5$;

$R^5$ représente un atome d'hydrogène ou un groupe alkyle en $C_1-C_{28}$, qui peut éventuellement être une ou plusieurs fois substitué par des radicaux, identiques ou différents, choisis dans l'ensemble constitué par des groupes hydroxyle, hydroxycarbonyle, aminocarbonyle, mono- ou dialkyl($C_1-C_{18}$)aminocarbonyle, aminoalkyl($C_2-C_{14}$)aminocarbonyle, aminoalkyl($C_1-C_3$)-phénylalkyl($C_1-C_3$)aminocarbonyle, alkyl($C_1-C_{18}$)-carbonyl-aminoalkyl($C_1-C_3$)-phényl-alkyl($C_1-C_3$)aminocarbonyle, alkyl($C_1-C_{18}$)-carbonylamino-alkyl($C_2-C_{14}$)amino-carbonyle, phényl-alcoxy($C_1-C_8$)-carbonyle qui peut également être substitué sur le fragment aryle, amino, mercapto, alcoxy en $C_1-C_{18}$, alcoxy($C_1-C_{18}$)-carbonyle, cycloalkyle en $C_3-C_8$ éventuellement substitué, des atomes d'halogène, les groupes nitro, trifluorométhyle ou un radical $R^6$;

$R^6$ représentant un groupe aryle en $C_6-C_{14}$ éventuellement substitué, aryl($C_6-C_{14}$)-alkyle($C_1-C_8$) éventuellement substitué, ou un cycle hétérocyclique mono- ou bicyclique à 5-12 chaînons, qui peut être aromatique, partiellement hydrogéné ou totalement hydrogéné, et qui peut contenir comme hétéroatome(s) 1, 2 ou 3 atomes d'azote, d'oxygène ou de soufre, identiques ou différents, ou représente un radical $R^7$, le radical aryle et, indépendamment de celui-ci, le radical hétérocyclique, pouvant éventuellement être une ou plusieurs fois substitués par des substituants, identiques ou différents, choisis parmi des atomes d'halogène et des groupes alkyle en $C_1-C_{18}$, alcoxy en $C_1-C_{18}$, nitro, amino et trifluorométhyle;

$R^7$ représente un groupe $-NR^8R^9$, $-OR^8$, $-SR^8$, une chaîne latérale d'aminoacide, un reste d'aminoacide naturel ou non naturel, d'iminoacide, d'aza-aminoacide éventuellement substitué sur l'atome d'azote par un radical alkyle en $C_1-C_8$ ou aryl($C_6-C_{14}$)-alkyle($C_1-C_8$), ou de dipeptide qui peut également être substitué sur le fragment aryle et/ou dans lequel la liaison peptidique peut également être réduite en $-NH-CH_2-$, ainsi que de leurs esters et amides, des groupes fonctionnels libres pouvant éventuellement être substitués par un atome d'hydrogène ou par le groupe hydroxyméthyle ou être protégés par des groupes protecteurs usuels dans la chimie des peptides, ou représente un radical $-COR^{7'}$, dans lequel $R^{7'}$ est défini comme $R^7$;

$R^8$ représente un atome d'hydrogène ou un groupe alkyle en $C_2-C_{18}$, aryl($C_6-C_{14}$)-alkyle($C_1-C_8$) éventuel-

lement substitué, alkyl($C_1$-$C_{18}$)-carbonyle, alcoxy($C_1$-$C_{18}$)-carbonyle, aryl($C_6$-$C_{14}$)-carbonyle, aryl($C_6$-$C_{14}$)-alkyl($C_1$-$C_8$)-carbonyle ou aryl($C_6$-$C_{14}$)-alcoxy($C_1$-$C_{18}$)-carbonyle, les groupes alkyle pouvant éventuellement être substitués par un groupe amino et/ou les radicaux aryle pouvant être substitués une ou plusieurs fois, de préférence une fois, par des substituants identiques ou différents, choisis parmi des atomes d'halogène et des groupes alkyle en $C_1$-$C_8$, alcoxy en $C_1$-$C_8$, nitro, amino et trifluorométhyle, ou représente un reste d'aminoacide naturel ou non naturel, d'iminoacide, d'aza-aminoacide éventuellement substitué sur l'atome d'azote par un radical alkyle en $C_1$-$C_8$ ou aryl($C_6$-$C_{14}$)alkyle($C_1$-$C_8$), ou de dipeptide qui peut également être substitué sur le fragment aryle et/ou dans lequel la liaison peptidique peut également être réduite en $-NH-CH_2-$; et

$R^9$ représente un atome d'hydrogène ou un groupe alkyle en $C_1$-$C_{18}$, un groupe aryle en $C_6$-$C_{14}$ éventuellement substitué, ou aryl($C_6$-$C_{14}$)-alkyle($C_1$-$C_8$) qui peut également être substitué sur le fragment aryle;

ainsi que leurs sels physiologiquement acceptables.

**2.** Dérivés de phénylimidazolidine selon la revendication 1, caractérisés en ce que

Y représente $-(CH_2)_m-CO-$ m représentant 1 ou 2, ou

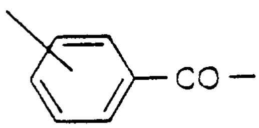

r est égal à 1;
Z représente un atome d'oxygène ou de soufre;
W représente un groupe hydroxyle, alcoxy en $C_1$-$C_4$, en particulier méthoxy, éthoxy ou 2-propyloxy;
R représente un atome d'hydrogène;
$R^1$ représente $-NH-C(=NH)-NH_2$, $-C(=NH)-NH_2$ ou $-CH_2-NH_2$ ou leurs dérivés méthoxycarbonyle;
$R^2$ représente un atome d'hydrogène ou le groupe méthyle;
$R^3$ représente un atome d'hydrogène; et
$R^4$ représente $-CO-NH-R^5$, $-NH-R^5$ représentant un reste d'α-aminoacide ou un x-amino-alkyl($C_2$-$C_8$)amide de celui-ci.

**3.** Dérivés de phénylimidazolidine selon la revendication 1 et/ou la revendication 2, caractérisés en ce que $R^4$ représente un groupe $-CO-NH-R^5$, $R^5$ représentant le reste valine, lysine, phénylalanine ou phénylglycine.

**4.** Dérivés de phénylimidazolidine selon une ou plusieurs des revendications 1 à 3, caractérisés en ce que $R^4$ représente un groupe $-CO-NH-R^5$, $R^5$ représentant le fragment 4-aminobutylamide d'un α-aminoacide.

**5.** Procédé pour la préparation des dérivés de phénylimidazolidine de formule I selon une ou plusieurs des revendications 1 à 4, caractérisé en ce que l'on effectue une condensation de fragments, en condensant un composé de formule générale III

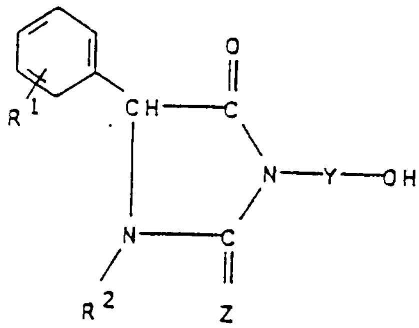

(III)

avec un composé de formule générale IV

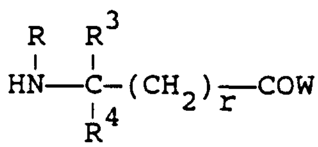

(IV)

r et les radicaux R, $R^1$ à $R^4$, Y, Z et W ayant les définitions données dans une ou plusieurs des revendications 1 à 4.

6. Composé de formule générale I selon une ou plusieurs des revendication 1 à 4, ainsi que leurs sels physiologiquement acceptables, pour utilisation en tant qu'inhibiteurs de l'agrégation des thrombocytes, de thromboses, de la dissémination de métastases de cellules cancéreuses ou de la fixation d'ostéoclastes à la surface des os.

7. Composition pharmaceutique, caractérisée en ce qu'elle contient, en tant que substance active, un ou plusieurs des composés de formule générale I selon une ou plusieurs des revendications 1 à 4, ou un(des) sel(s) physiologiquement acceptable(s) de celui-ci(ceux-ci), conjointement avec des additifs et véhicules pharmaceutiquement acceptables et éventuellement encore une ou plusieurs autres substances pharmacologiquement actives.

8. Procédé pour la préparation d'une composition pharmaceutique, contenant un ou plusieurs des composés de formule générale I selon une ou plusieurs des revendications 1 à 4, ou un(des) sel(s) physiologiquement acceptable(s) de celui-ci (ceux-ci), caractérisé en ce qu'on le(s) met sous une forme d'administration appropriée, conjointement avec des additifs et véhicules pharmaceutiquement acceptables et éventuellement encore une ou plusieurs autres substances pharmacologiquement actives.